# EUROPEAN PATENT APPLICATION

(11) **EP 4 295 905 A1**
(43) Date of publication of application: **27.12.2023**
(21) Application number: 23193515.6
(22) Date of filing: 08.07.2021
(51) Int. Cl.: A61P 9/00, C07K 16/18, A61K 39/00

(54) **DOSAGE AND ADMINISTRATION OF ANTI-C5 ANTIBODIES FOR TREATING PAROXYSMAL NOCTURNAL HEMOGLOBINURIA (PNH) IN PEDIATRIC PATIENTS**

(30) Priority: 09.07.2020 US 202063049768 P
(62) Divisional of application: 21748759.4
(71) Applicant: Alexion Pharmaceuticals, Inc., Boston, MA 02210 (US)
(72) Inventor: PAYTON, Lori, Madison, 06443 (US); ROTTINGHAUS, Scott, T., Boston, 02210 (US); PRADHAN, Rajendra, Boston, 02210 (US); ORTIZ, Stephan, Boston, 02210 (US); OGAWA, Masayo, Boston, 02210 (US); GAO, Xiang, Boston, 02210 (US)
(74) Representative: J A Kemp LLP

(57) **Abstract**

Provided are methods for clinical treatment of Paroxysmal Nocturnal Hemoglobinuria (PNH) in pediatric patients using an anti-C5 antibody, or antigen binding fragment thereof.

## Description

### CROSS REFERENCE TO RELATED APPLICATIONS

This application claims the benefit of U.S. Provisional Application No. 63/049,768, filed July 9, 2020, the contents of which is incorporated by reference herein in its entirety.

### BACKGROUND

Paroxysmal nocturnal hemoglobinuria (PNH) is a progressive, debilitating, and life-threatening disease characterized by complement-mediated hemolysis, thrombosis and bone marrow failure. PNH has an estimated worldwide incidence of 1.3 per million population. The onset of PNH is typically in adulthood, with pediatric cases accounting for <5% of reported cases. Given the extremely small target population, studies of children with PNH have been limited to case reports, case series, and a small clinical trial.

In adults, the clinical manifestations of PNH include hemoglobinuria, chronic renal insufficiency, erectile dysfunction, thrombosis, abdominal pain, dyspnea and dysphagia. In contrast, children with PNH usually present with nonspecific symptoms related to the underlying bone marrow disorder, such as pallor, fatigue or jaundice, with hemoglobinuria appearing less commonly. Clinical evaluation in pediatric patients also reveals bone marrow failure syndromes, such as aplastic anemia and refractory cytopenia. Once the bone marrow disorder is resolved in the child or the PNH clone expands (the cause of which is still unknown), the disease eventually evolves into one more typically seen in adults at presentation.

Thus, pediatric patients can be expected to suffer substantial morbidity related to hemolysis, as seen in adult PNH patients. Accordingly, it is an object of the present disclosure to provide improved methods for treating patients with PNH.

### SUMMARY

Provided herein are compositions and methods for treating Paroxysmal Nocturnal Hemoglobinuria (PNH) in a human pediatric patient, comprising administering to the patient an anti-C5 antibody or antigen binding fragment thereof, wherein the anti-C5 antibody or antigen binding fragment thereof is administered (or is for administration) according to a particular clinical dosage regimen (e.g., at a particular dose amount and according to a specific dosing schedule).

An exemplary anti-C5 antibody is ravulizumab (ULTOMIRIS^{®}) comprising the heavy and light chains having the sequences shown in SEQ ID NOs:14 and 11, respectively, or antigen binding fragments and variants thereof. In other embodiments, the antibody comprises the heavy and light chain complementarity determining regions (CDRs) or variable regions (VRs) of ravulizumab. Accordingly, in one embodiment, the antibody comprises the CDR1, CDR2 and CDR3 domains of the heavy chain variable (VH) region of ravulizumab having the sequence shown in SEQ ID NO:12, and the CDR1, CDR2 and CDR3 domains of the light chain variable (VL) region of ravulizumab having the sequence shown in SEQ ID NO:8. In another embodiment, the antibody comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively. In another embodiment, the antibody comprises VH and VL regions having the amino acid sequences set forth in SEQ ID NO:12 and SEQ ID NO:8, respectively. In another embodiment, the antibody comprises a heavy chain constant region as set forth in SEQ ID NO:13.

In another embodiment, the antibody comprises a variant human Fc constant region that binds to human neonatal Fc receptor (FcRn), wherein the variant human Fc CH3 constant region comprises Met429Leu and Asn435Ser substitutions at residues corresponding to methionine 428 and asparagine 434 of a native human IgG Fc constant region, each according to the EU numbering convention.

In another embodiment, the antibody comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively and a variant human Fc constant region that binds to human neonatal Fc receptor (FcRn), wherein the variant human Fc CH3 constant region comprises Met429Leu and Asn435Ser substitutions at residues corresponding to methionine 428 and asparagine 434 of a native human IgG Fc constant region, each according to the EU numbering convention.

In another embodiment, the anti-C5 antibody comprises the heavy and light chain CDRs or variable regions of the BNJ421 antibody (described in WO2015134894 and US Patent No. 9,079,949). In another embodiment, the anti-C5 antibody comprises the heavy and light chain CDRs or variable regions of the 7086 antibody (see US Patent Nos. 8,241,628 and 8,883,158). In another embodiment, the anti-C5 antibody comprises the heavy and light chain CDRs or variable regions of the 8110 antibody (see US Patent Nos. 8,241,628 and 8,883,158). In another embodiment, the anti-C5 antibody comprises the heavy and light chain CDRs or variable regions of the 305LO5 antibody (see US Patent No. 9,765,135). In another embodiment, the anti-C5 antibody comprises the heavy and light chain CDRs or variable regions of the SKY59 antibody. In another embodiment, the anti-C5 antibody comprises the heavy and light chain CDRs or variable regions of the REGN3918 antibody.

In another embodiment, the antibody competes for binding with, and/or binds to the same epitope on C5 as any of the above-mentioned antibodies. In another embodiment, the antibody has at least about 90% variable region amino acid sequence identity to any of the above-mentioned antibodies (*e*.*g*., at least about 90%, 95% or 99% variable region identity with SEQ ID NO: 12 or SEQ ID NO: 8).

In another embodiment, the antibody binds to human C5 at pH 7.4 and 25C with an affinity dissociation constant (K_{D}) that is in the range 0.1 nM ≤ K_{D} ≤ 1 nM. In another embodiment, the antibody binds to human C5 at pH 6.0 and 25C with a K_{D} ≥ 10 nM. In yet another embodiment, the [(K_{D} of the antibody or antigen-binding fragment thereof for human C5 at pH 6.0 and at 25C)/(K_{D} of the antibody or antigen-binding fragment thereof for human C5 at pH 7.4 and at 25C)] of the antibody is greater than 25.

In one embodiment, the dose of the anti-C5 antibody, or antigen binding fragment thereof, is based on the weight of the patient. In one embodiment, for example, 300 mg of the anti-C5 antibody or antigen binding fragment thereof is administered to a patient weighing ≥ 5 to < 10 kg. In another embodiment, 600 mg of the anti-C5 antibody or antigen binding fragment thereof is administered to a patient weighing ≥ 10 to < 20 kg. In another embodiment, 900 mg or 2100 mg of the anti-C5 antibody or antigen binding fragment thereof is administered to a patient weighing ≥ 20 to < 30 kg. In another embodiment, 1200 mg or 2700 mg of the anti-C5 antibody or antigen binding fragment thereof is administered to a patient weighing ≥ 30 to < 40 kg. In another embodiment, 2400 mg or 3000 mg of the anti-C5 antibody or antigen binding fragment thereof is administered to a patient weighing ≥ 40 to < 60 kg. In another embodiment, 2700 mg or 3300 mg of the anti-C5 antibody or antigen binding fragment thereof is administered to a patient weighing > 60 to < 100 kg. In another embodiment, 3000 mg or 3600 mg of the anti-C5 antibody or antigen binding fragment thereof is administered to a patient weighing > 100 kg. In certain embodiments, dosage regimens are adjusted to provide the optimum desired response (*e*.*g*., an effective response).

In another embodiment, the anti-C5 antibody or antigen binding fragment thereof is administered for one or more administration cycles. In one embodiment, the treatment (*e*.*g*., administration cycle) is 26 weeks. In one embodiment, the anti-C5 antibody or antigen binding fragment thereof is administered once on Day 1 (*e*.*g*., of the administration cycle), once on Day 15 *(*e*.g.,* of the administration cycle), and every four weeks thereafter. In another embodiment, the anti-C5 antibody or antigen binding fragment thereof is administered once on Day 1 (*e.g.,* of the administration cycle), once on Day 15 (*e.g.,* of the administration cycle), and every eight weeks thereafter. In another embodiment, the anti-C5 antibody or antigen binding fragment thereof is administered every four or eight weeks after treatment (*e.g.,* an administration cycle) for an extension period up to two years (*e.g.,* at a dose of 300 mg, 600 mg, 900 mg, 1200 mg, 2100 mg, 2400 mg, 2700 mg, 3000 mg, 3300 mg or 3600 mg).

In another embodiment, a method of treating a human patient with PNH is provided, the method comprising administering to the patient (*e.g.,* during an administration cycle) an effective amount of an anti-C5 antibody or antigen binding fragment thereof, comprising CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively, wherein the anti-C5 antibody or antigen binding fragment thereof is administered: (a) once on Day 1 at a dose of 600 mg to a patient weighing > 5 to < 10 kg, 600 mg to a patient weighing > 10 to < 20 kg, 900 mg to a patient weighing > 20 to < 30 kg, 1200 mg to a patient weighing > 30 to < 40 kg, 2400 mg to a patient weighing ≥ 40 to < 60 kg, 2700 mg to a patient weighing > 60 to < 100 kg, or 3000 mg to a patient weighing ≥ 100 kg; and (b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing > 100 kg.

In another embodiment, a method of treating a human patient with PNH is provided, the method comprising administering to the patient (*e.g.,* during an administration cycle) an effective amount of an anti-C5 antibody or antigen binding fragment thereof, comprising CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively, wherein the anti-C5 antibody or antigen binding fragment thereof is administered: (a) once on Day 1 at a dose of 600 mg to a patient weighing > 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and (b) on Day 15 and every four weeks thereafter at a dose of 300 mg to a patient weighing ≥ 5 to < 10 kg or 600 mg to a patient weighing > 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing > 100 kg, and wherein the treatment results in a reduction in LDH levels to within normal levels or to within 50% below what is considered the ULN level (*e*.*g*., within 105-333 IU/L (international units per liter), a serum trough concentration of the anti-C5 antibody, or antigen binding fragment thereof, of at least 175 µg/mL or greater, and/or a free C5 concentration of 0.5 µg/mL or less (*e.g.,* 0.4 µg/mL, 0.3 µg/mL, 0.2 µg/mL, or 0.1 µg/mL or less).

In another embodiments, the treatment results in a reduction of at least one clinical parameter selected from (a) lactate dehydrogenase (LDH) percent change from baseline (LDH-PCHG); (b) transfusion avoidance (TA); (c) pediatric FACIT Fatigue (FACIT); (d) hemoglobin stabilization (HGB-S); (e) free hemoglobin percent change from baseline (Free HGB-PCHG); and (1) breakthrough hemolysis (BTH) or a combination thereof; preferably wherein the treatment results in reduction in breakthrough hemolysis (BTH). In another embodiment, the treatment eliminates breakthrough hemolysis (BTH).

In another embodiment, a method of treating a human patient with PNH is provided, the method comprising administering to the patient (e.g., during an administration cycle) an effective amount of an anti-C5 antibody or antigen binding fragment thereof comprising CDR1, CDR2 and CDR3 heavy chain sequences as set forth in SEQ ID NOs:19, 18 and 3, respectively, CDR1, CDR2 and CDR3 light chain sequences as set forth in SEQ ID NOs:4, 5 and 6, respectively, and a variant human Fc constant region that binds to human neonatal Fc receptor (FcRn), wherein the variant human Fc CH3 constant region comprises Met429Leu and Asn435Ser substitutions at residues corresponding to methionine 428 and asparagine 434 of a native human IgG Fc constant region, each according to the EU numbering convention, wherein the anti-C5 antibody or antigen binding fragment thereof is administered: (a) once on Day 1 at a dose of 600 mg to a patient weighing ≥ 5 to < 10 kg, 600 mg to a patient weighing ≥ 10 to < 20 kg, 900 mg to a patient weighing > 20 to < 30 kg, 1200 mg to a patient weighing ≥ 30 to < 40 kg, 2400 mg to a patient weighing > 40 to < 60 kg, 2700 mg to a patient weighing ≥ 60 to < 100 kg, or 3000 mg to a patient weighing ≥ 100 kg; and (b) on Day 15 and every four weeks thereafter at a dose of 300 mg to a patient weighing ≥ 5 to < 10 kg or 600 mg to a patient weighing > 10 to < 20 kg; or on Day 15 and every eight weeks thereafter at a dose of 2100 mg to a patient weighing > 20 to < 30 kg, 2700 mg to a patient weighing ≥ 30 to < 40 kg, 3000 mg to a patient weighing > 40 to < 60 kg, 3300 mg to a patient weighing > 60 to < 100 kg, or 3600 mg to a patient weighing > 100 kg.

In another embodiment, the anti-C5 antibody or antigen binding fragment thereof is administered to a patient weighing ≥ 5 to < 10 kg: (a) once on Day 1 at a dose of 600 mg; and (b) on Day 15 and every four weeks thereafter at a dose of 300 mg.

In another embodiment, the anti-C5 antibody is administered to a patient weighing ≥ 10 to < 20 kg: (a) once on Day 1 at a dose of 600 mg; and (b) on Day 15 and every four weeks thereafter at a dose of 600 mg.

In another embodiment, the anti-C5 antibody is administered to a patient weighing ≥ 20 to < 30 kg: (a) once on Day 1 at a dose of 900 mg; and (b) on Day 15 and every eight weeks thereafter at a dose of 2100 mg.

In another embodiment, the anti-C5 antibody is administered to a patient weighing ≥ 30 to < 40 kg: (a) once on Day 1 at a dose of 1200 mg; and (b) on Day 15 and every eight weeks thereafter at a dose of 2700 mg.

In another embodiment, the anti-C5 antibody is administered to a patient weighing ≥ 40 to < 60 kg: (a) once on Day 1 at a dose of 2400 mg; and (b) on Day 15 and every eight weeks thereafter at a dose of 3000 mg.

In another embodiment, the anti-C5 antibody is administered to a patient weighing ≥ 60 to < 100 kg: (a) once on Day 1 at a dose of 2700 mg; and (b) on Day 15 and every eight weeks thereafter at a dose of 3300 mg.

In another embodiment, the anti-C5 antibody is administered to a patient weighing ≥ 100 kg: (a) once on Day 1 at a dose of 3000 mg; and (b) on Day 15 and every eight weeks thereafter at a dose of 3600 mg.

In one embodiment, the patient has not previously been treated with eculizumab. In another embodiment, the patient has previously been treated with eculizumab. In another embodiment, the patient has previously been treated with eculizumab and Day 1 (*e*.*g*., of the administration cycle) is two weeks or more from the patient's last dose of eculizumab.

In another aspect, the treatment regimens described are sufficient to maintain particular serum trough concentrations of the anti-C5 antibody or antigen binding fragment thereof. In one embodiment, for example, the treatment regimen maintains a serum trough concentration of the anti-C5 antibody or antigen binding fragment thereof of 50, 55, 60, 65, 70, 75, 80, 85, 90, 95, 100, 105, 110, 115, 120, 125, 130, 135, 140, 145, 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395 or 400 µg/mL or greater. In one embodiment, the treatment regimen maintains a serum trough concentration of the anti-C5 antibody or antigen binding fragment thereof of 100 µg/mL or greater, 150 µg/mL or greater, 200 µg/mL or greater, 250 µg/mL or greater, or 300 µg/mL or greater. In another embodiment, the treatment maintains a serum trough concentration of the anti-C5 antibody or antigen binding fragment thereof of between 100 µg/mL and 200 µg/mL. In another embodiment, the treatment maintains a serum trough concentration of the anti-C5 antibody or antigen binding fragment thereof of about 175 µg/mL.

In another embodiment, to obtain an effective response, the anti-C5 antibody is administered to the patient in an amount and with a frequency to maintain at least 50 µg, 55 µg, 60 µg, 65 µg, 70 µg, 75 µg, 80 µg, 85 µg, 90 µg, 95 µg, 100 µg, 105 µg, 110 µg, 115 µg, 120 µg, 125 µg, 130 µg, 135 µg, 140 µg, 145 µg, 150 µg, 155 µg, 160 µg, 165 µg, 170 µg, 175 µg. 180 µg, 185 µg, 190 µg, 195 µg, 200 µg, 205 µg, 210 µg, 215 µg, 220 µg, 225 µg, 230 µg, 235 µg, 240 µg, 245 µg, 250 µg, 255 µg or 260 µg of antibody per milliliter of the patient's blood. In another embodiment, the anti-C5 antibody is administered to the patient in an amount and with a frequency to maintain between 50 µg and 250 µg of antibody per milliliter of the patient's blood. In another embodiment, the anti-C5 antibody is administered to the patient in an amount and with a frequency to maintain between 100 µg and 200 µg of antibody per milliliter of the patient's blood. In another embodiment, the anti-C5 antibody is administered to the patient in an amount and with a frequency to maintain about 175 µg of antibody per milliliter of the patient's blood.

In another embodiment, to obtain an effective response, the anti-C5 antibody is administered to the patient in an amount and with a frequency to maintain a minimum free C5 concentration. In one embodiment, for example, the anti-C5 antibody is administered to the patient in an amount and with a frequency to maintain a free C5 concentration of 0.5 µg/mL or less (*e.g.,* 0.4 µg/mL, 0.3 µg/mL, 0.2 µg/mL, or 0.1 µg/mL or less).

The anti-C5 antibodies, or antigen binding fragments thereof, can be administered to a patient by any suitable means. In one embodiment, the antibodies are formulated for intravenous administration.

The efficacy of the treatment methods provided herein can be assessed using any suitable means. In one embodiment, for a pediatric PNH patient, the treatment produces at least one therapeutic effect selected from the group consisting of: a reduction or cessation in fatigue, abdominal pain, dyspnea, dysphagia and chest pain compared to baseline. In another embodiment, the treatment results in terminal complement inhibition. In another embodiment, the treatment results in a reduction of hemolysis as assessed by lactate dehydrogenase (LDH) levels compared to baseline. In another embodiment, the treatment produces a shift toward normal levels of at least one hemolysis-related hematologic biomarker selected, for example, from the group consisting of: free hemoglobin, haptoglobin, reticulocyte count, PNH red blood cell (RBC) clone and D-dimer. In another embodiment, the treatment produces a reduction in the need for blood transfusions compared to baseline. In another embodiment, the treatment produces a reduction in major adverse vascular events (MAVEs). In another embodiment, the treatment produces a shift toward normal levels of a chronic disease associated biomarker selected from the group consisting estimated glomerular filtration rate (eGFR) and spot urine:albumin:creatinine and plasma brain natriuretic peptide (BNP). In another embodiment, the treatment produces a change from baseline in quality of life as assessed via the Functional Assessment of Chronic Illness Therapy (FACIT)-Fatigue Scale, version 4 and the European Organisation for Research and Treatment of Cancer, Quality of Life Questionnaire-Core 30 Scale compared to baseline.

In a particular embodiment, LDH levels are used to evaluate responsiveness to a therapy (e.g., a reduction of hemolysis as assessed by LDH levels is indicative of an improvement in at least one sign of PNH). In one embodiment, patients treated according to the disclosed methods experience reductions in LDH levels to near normal levels or to within 10%, or within 20% above what is considered the normal level (*e*.*g*., within 105-333 IU/L (international units per liter). In another embodiment, the patient's LDH levels are normalized throughout maintenance period of treatment. In another embodiment, the treated patient's LDH levels are normalized at least at least 95% of the time while on the maintenance period of treatment. In another embodiment, the treated patient's LDH levels are normalized at least at least 90%, 85% or 80% of the time while on the maintenance period of treatment. In one embodiment, the patient's LDH levels are ≥ 1.5 fold above the upper limit of normal (LDH ≥ 1.5 × ULN) prior to initiating treatment.

In one embodiment, patients treated according to the disclosed methods experience reductions in LDH levels to within normal levels or to within 10%, 11%, 12%, 13%, 14%, 15%, 16%, 17%, 18%, 19%, 20%, 21%, 22%, 23%, 24%, 25%, 26%, 28%, 29%, 30%, 31%, 32%, 33%, 34%, 35%, 36%, 37%, 38%, 39%, 40%, 41%, 42%, 43%,44%, 45%, 46%, 47%, 48%, 49% or within 50% below what is considered the ULN level (*e*.*g*., within 105-333 IU/L (international units per liter). In one embodiment, the patient's LDH levels are ≥ 1.5 fold above the ULN (LDH ≥ 1.5 × ULN) prior to initiating treatment.

In one embodiment, patients treated according to the disclosed methods experience an LDH percent change compared to baseline of 10%, 11%, 12%, 13%, 14%, 15%, 16%, 17%, 18%, 19%, 20%, 21%, 22%, 23%, 24%, 25%, 26%, 27%, 28%, 29%, 30%. 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53% 54%, 55%, 56%, 57%, 58%, 59%, or 60%.

In one embodiment, patients treated according to the disclosed methods maintain a serum trough concentration of the anti-C5 antibody, or antigen binding fragment thereof, of at least 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395 or 400 µg/mL or greater. In one embodiment, patients treated according to the disclosed methods maintain a serum trough concentration of the anti-C5 antibody, or antigen binding fragment thereof, of at least 175 µg/mL or greater.

In one embodiment, patients treated according to the disclosed methods have a free C5 concentration of 0.5 µg/mL or less (*e.g.,* 0.4 µg/mL, 0.3 µg/mL, 0.2 µg/mL, or 0.1 µg/mL or less).

In one embodiment, patients treated according to the disclosed methods do not experience breakthrough hemolysis (BTH).

In another aspect, an anti-C5 antibody or antigen binding fragment thereof is provided, comprising CDR1, CDR2 and CDR3 domains of the heavy chain variable region having the sequence set forth in SEQ ID NO:12, and CDR1, CDR2 and CDR3 domains of the light chain variable region having the sequence set forth in SEQ ID NO:8, for administration: (a) once on Day 1 at a dose of 600 mg to a patient weighing > 5 to < 10 kg, 600 mg to a patient weighing ≥ 10 to < 20 kg, 900 mg to a patient weighing > 20 to < 30 kg, 1200 mg to a patient weighing ≥ 30 to < 40 kg, 2400 mg to a patient weighing > 40 to < 60 kg, 2700 mg to a patient weighing ≥ 60 to < 100 kg, or 3000 mg to a patient weighing > 100 kg; and (b) on Day 15 and every four weeks thereafter at a dose of 300 mg to a patient weighing ≥ 5 to < 10 kg or 600 mg to a patient weighing > 10 to < 20 kg; or on Day 15 and every eight weeks thereafter at a dose of 2100 mg to a patient weighing > 20 to < 30 kg, 2700 mg to a patient weighing ≥ 30 to < 40 kg, 3000 mg to a patient weighing > 40 to < 60 kg, 3300 mg to a patient weighing > 60 to < 100 kg, or 3600 mg to a patient weighing > 100 kg.

In one embodiment, the antibody is determined to be safe, tolerable and sufficiently non-immunogenic after multiple IV doses for use in PNH pediatric patients.

Further provided are kits that include a pharmaceutical composition containing an anti-C5 antibody or antigen binding fragment thereof, such as ravulizumab, and a pharmaceutically acceptable carrier, in a therapeutically effective amount adapted for use in the methods described herein. In one embodiment, the kit comprises: (a) a dose of an anti-C5 antibody or antigen binding fragment thereof, comprising CDR1, CDR2 and CDR3 domains of the heavy chain variable region having the sequence set forth in SEQ ID NO: 12, and CDR1, CDR2 and CDR3 domains of the light chain variable region having the sequence set forth in SEQ ID NO:8; and (b) instructions for using the anti-C5 antibody or antigen binding fragment thereof in the methods described herein.

In one embodiment, 300 mg or 600 mg of the anti-C5 antibody or antigen binding fragment thereof is administered to a patient weighing ≥ 5 to < 10 kg. In another embodiment, 600 mg of the anti-C5 antibody or antigen binding fragment thereof is administered to a patient weighing ≥ 10 to < 20 kg. In another embodiment, 900 mg or 2100 mg of the anti-C5 antibody or antigen binding fragment thereof is administered to a patient weighing ≥ 20 to < 30 kg. In another embodiment, 1200 mg or 2700 mg of the anti-C5 antibody or antigen binding fragment thereof is administered to a patient weighing ≥ 30 to < 40 kg. In another embodiment, 2400 mg or 3000 mg of the anti-C5 antibody or antigen binding fragment thereof is administered to a patient weighing > 40 to < 60 kg. In another embodiment, 2700 mg or 3300 mg of the anti-C5 antibody or antigen binding fragment thereof is administered to a patient weighing > 60 to < 100 kg. In another embodiment, 3000 mg or 3600 mg of the anti-C5 antibody or antigen binding fragment thereof is administered to a patient weighing ≥ 100 kg.

### BRIEF DESCRIPTION OF THE DRAWINGS

**FIG. 1** is a schematic depicting the overall study design.
**FIG. 2** is a schematic depicting the overall patient disposition of the study. Thirteen patients were enrolled and twelve were included in the interim analysis. No patients discontinued in the primary evaluation period.
**FIG. 3** sets forth the baseline patient demographics.
**FIG. 4** sets forth the baseline disease characteristics.
**FIG. 5** sets forth ravulizumab serum concentrations over time for treatment naïve and eculizumab experienced patients.
**FIG. 6** is a graph depicting mean (SD) ravulizumab serum concentrations (linear scale) over time for treatment naive and eculizumab experienced patients.
**FIG. 7** sets forth free C5 and CRBC results over time for treatment naive and eculizumab experienced patients.
**FIG. 8** is a graph depicting mean (95% CI) cRBC over time for treatment naive and eculizumab experienced patients. The dashed line of 20% hemolysis represents a discrete (qualitative) threshold for complete terminal complement inhibition.
**FIG. 9** is a graph depicting mean (95% CI) free C5 over time for treatment naive and eculizumab experienced patients. The dashed line for serum free C5 values of 0.5 µg/mL represents a threshold for complete terminal complement inhibition.
**FIG. 10** is a graph depicting mean (95% CI) change from baseline in pediatric FACIT Fatigue Score over time for treatment naive and eculizumab experienced patients. The dashed line of FACIT Fatigue Score of 3 represents threshold level for clinically-meaningful results.
**FIG. 11** is a graph depicting mean (95% CI) pediatric FACIT Fatigue Score over time for treatment naive and eculizumab experienced patients.
**FIG. 12** summarizes the overall efficacy results, including the following endpoints: LDH Percent Change from Baseline (LDH-PCHG), Transfusion Avoidance (TA), pediatric FACIT Fatigue (FACIT); Hemoglobin Stabilization (HGB-S), Free Hemoglobin Percent Change from Baseline (Free HGB-PCHG), and Breakthrough Hemolysis (BTH).
**FIG. 13** is a graph depicting mean (95% CI) % change in LDH over time for treatment naive and eculizumab experienced patients.
**FIG. 14** is a graph depicting mean (95% CI) LDH (U/L) over time for treatment naive and eculizumab experienced patients.
**FIG. 15** provides an overview of key safety points.
**FIG. 16** provides an overview of treatment-emergent serious adverse events (TESAES).

### DETAILED DESCRIPTION

### I. Definitions

As used herein, the term "subject" or "patient" is a human patient (*e*.*g*., a patient having Paroxysmal Nocturnal Hemoglobinuria (PNH)).

As used herein, the term "pediatric" patient is a human patient under 18 years of age (<18 years of age).

PNH is an acquired hemolytic disorder that occurs most frequently in adults (Brodsky, R., Blood, 126:2459-65, 2015). The disease begins with the clonal expansion of a hematopoietic stem cell that has acquired a somatic mutation in the PIGA gene (Brodsky, R., Blood, 124:2804-11, 2014). Consequently, PNH blood cells lack the glycophosphatidylinositol (GPI) anchor protein and are deficient in the membrane-bound complement inhibitory proteins CD55 and CD59. In the absence of CD55, there is increased deposition of complement protein C3 cleavage products on blood cell membrane surfaces, in turn leading to cleavage of C5 into C5a and C5b. The pathology and clinical presentations in patients with PNH are driven by uncontrolled terminal complement activation.

C5a is a potent anaphylatoxin, chemotactic factor, and cell-activating molecule that mediates multiple pro-inflammatory and pro-thrombotic activities (Matis, L & Rollins, S., Nat. Med.,1:839-42, 1995; Prodinger et al., Complement. In: Paul WE, editor. Fundamental immunology (4th ed). Philadelphia: Lippincott-Raven Publishers; 1999. p. 967-95). C5b recruits the terminal complement components C6, C7, C8 and C9 to form the pro-inflammatory, pro-thrombotic cytolytic pore molecule C5b-9, a process that under normal circumstances would be blocked on the red blood cell (RBC) membrane by CD59. In patients with PNH, however, these final steps proceed unchecked, culminating in hemolysis and the release of free hemoglobin, as well as platelet activation (Hill, A. et al., Blood, 121:4985-96, 2013). The signs and symptoms of PNH can be attributed to chronic, uncontrolled complement C5 cleavage, and release of C5a and C5b-9 leading to RBC hemolysis, which together result in:
- release of intracellular free hemoglobin and lactate dehydrogenase (LDH) into circulation as a direct consequence of hemolysis;
- irreversible binding to and inactivation of nitric oxide (NO) by hemoglobin, and inhibition of NO synthesis;
- vasoconstriction and tissue-bed ischemia due to absence of vasodilatory NO, as well as possible microthrombi manifesting as abdominal pain, dysphagia and erectile dysfunction;
- platelet activation; and
- a pro-inflammatory and prothrombotic state.

A substantial proportion of patients with PNH experience renal dysfunction and pulmonary hypertension (Hillmen, P. et al., Am. J. Hematol., 85:553-9, 2010 [erratum in Am. J. Hematol., 85:911, 2010]; Hill, A. et al., Br. J. Haematol., 158:409-14, 2012). Patients also experience venous or arterial thrombosis in diverse sites, including the abdomen or central nervous system.

In contrast, children with PNH usually present with nonspecific symptoms related to the underlying bone marrow disorder, such as pallor, fatigue or jaundice, with hemoglobinuria appearing less commonly (Ware, R. et al., N. Engl. J. Med., 325:991-6, 1991). Clinical evaluation in pediatric patients also reveals bone marrow failure syndromes, such as, for example, aplastic anemia and refractory cytopenia (van den Heuvel-Eibrink, M., Paediatr. Drugs, 9:11-6, 2007). Once the bone marrow disorder is resolved in the child or the PNH clone expands (the cause of which is still unknown), the disease eventually evolves into one more typically seen in adults at presentation.

As used herein, "effective treatment" refers to treatment producing a beneficial effect, e.g., amelioration of at least one symptom of a disease or disorder. A beneficial effect can take the form of an improvement over baseline, *e.g.,* an improvement over a measurement or observation made prior to initiation of therapy according to the method. Effective treatment may refer to alleviation of at least one symptom of PNH (*e.g.,* pallor, fatigue, jaundice, anemia, cytopenia, abdominal pain, dyspnea, dysphagia, chest pain or erectile dysfunction).

The term "effective amount" refers to an amount of an agent that provides the desired biological, therapeutic and/or prophylactic result. That result can be reduction, amelioration, palliation, lessening, delaying and/or alleviation of one or more of the signs, symptoms or causes of a disease, or any other desired alteration of a biological system. In one example, an "effective amount" is the amount of anti-C5 antibody, or antigen binding fragment thereof, clinically proven to alleviate at least one symptom of PNH (*e.g.,* pallor, fatigue, jaundice, anemia, cytopenia, abdominal pain, dyspnea, dysphagia, or chest pain). An effective amount can be administered in one or more administrations.

As used herein, the term "loading dose" refers to the first dose administered (*e.g.,* during an administration cycle).

As used herein, the terms "maintenance" and "maintenance phase" are used interchangeably and refer to the second phase of treatment. In certain embodiments, treatment is continued as long as clinical benefit is observed or until unmanageable toxicity or disease progression occurs.

As used herein, the term "serum trough level" refers to the lowest level that the agent (*e.g.,* the anti-C5 antibody, or antigen binding fragment thereof) or medicine is present in the serum. In contrast, a "peak serum level," refers to the highest level of the agent in the serum. The "average serum level," refers to the mean level of the agent in the serum over time.

The term "antibody" describes a polypeptide comprising at least one antibody-derived antigen binding site (e.g., VH/VL region or Fv, or CDR). Antibodies include known forms of antibodies, e.g., the antibody can be a human antibody, a humanized antibody, a bispecific antibody or a chimeric antibody. The antibody also can be a Fab, Fab'2, ScFv, SMIP, Affibody^{®}, nanobody or a single-domain antibody. The antibody also can be of any of the following isotypes: IgG1, IgG2, IgG3, IgG4, IgM, IgA1, IgA2, IgAsec, IgD, IgE or combinations thereof. The antibody can be a naturally occurring antibody or an antibody that has been altered by a protein engineering technique (e.g., by mutation, deletion, substitution, conjugation to a non-antibody moiety). An antibody can include, for example, one or more variant amino acids (compared to a naturally occurring antibody) that change a property (*e.g.,* a functional property) of the antibody. Numerous such alterations are known in the art that affect, *e*.*g*., half-life, effector function, and/or immune responses to the antibody in a patient. The term antibody also includes artificial or engineered polypeptide constructs that comprise at least one antibody-derived antigen binding site.

### II. Anti-C5 Antibodies

Anti-C5 antibodies described herein bind to complement component C5 (*e.g.,* human C5) and inhibit the cleavage of C5 into fragments C5a and C5b. As described above, such antibodies also have, for example, improved pharmacokinetic properties relative to other anti-C5 antibodies (*e*.*g*., eculizumab) used for therapeutic purposes.

Anti-C5 antibodies (or VH/VL domains derived therefrom) suitable for use in the methods described herein can be generated using methods known in the art. Alternatively, art recognized anti-C5 antibodies can be used. Antibodies that compete for binding to C5 with any of these art recognized antibodies or antibodies described herein can also be used.

An exemplary anti-C5 antibody is ravulizumab comprising heavy and light chains having the sequences shown in SEQ ID NOs:14 and 11, respectively, or antigen binding fragments and variants thereof. Ravulizumab (also known as ULTOMIRIS^{®}, BNJ441 and ALXN1210) is described in WO2015134894 and US Patent No: 9,079,949, the entire teachings of which are hereby incorporated by reference. The terms ravulizumab, BNJ441, and ALXN1210 may be used interchangeably throughout this document, but all refer to the same antibody. Ravulizumab selectively binds to human complement protein C5, inhibiting its cleavage to C5a and C5b during complement activation. This inhibition prevents the release of the proinflammatory mediator C5a and the formation of the cytolytic pore-forming membrane attack complex (MAC) C5b-9 while preserving the proximal or early components of complement activation (e.g., C3 and C3b) essential for the opsonization of microorganisms and clearance of immune complexes.

In other embodiments, the antibody comprises the heavy and light chain CDRs or variable regions of ravulizumab. Accordingly, in one embodiment, the antibody comprises the CDR1, CDR2 and CDR3 domains of the VH region of ravulizumab having the sequence set forth in SEQ ID NO:12, and the CDR1, CDR2 and CDR3 domains of the VL region of ravulizumab having the sequence set forth in SEQ ID NO:8. In another embodiment, the antibody comprises heavy chain CDR1, CDR2 and CDR3 domains having the sequences set forth in SEQ ID NOs:19, 18 and 3, respectively, and light chain CDR1, CDR2 and CDR3 domains having the sequences set forth in SEQ ID NOs:4, 5 and 6, respectively. In another embodiment, the antibody comprises VH and VL regions having the amino acid sequences set forth in SEQ ID NO:12 and SEQ ID NO:8, respectively.

Another exemplary anti-C5 antibody is antibody BNJ421 comprising heavy and light chains having the sequences shown in SEQ ID NOs:20 and 11, respectively, or antigen binding fragments and variants thereof. BNJ421 (also known as ALXN1211) is described in WO2015134894 and US Patent No.9,079,949, the entire teachings of which are hereby incorporated by reference.

In other embodiments, the antibody comprises the heavy and light chain CDRs or variable regions of BNJ421. Accordingly, in one embodiment, the antibody comprises the CDR1, CDR2 and CDR3 domains of the VH region of BNJ421 having the sequence set forth in SEQ ID NO:12, and the CDR1, CDR2 and CDR3 domains of the VL region of BNJ421 having the sequence set forth in SEQ ID NO:8. In another embodiment, the antibody comprises heavy chain CDR1, CDR2 and CDR3 domains having the sequences set forth in SEQ ID NOs:19, 18 and 3, respectively, and light chain CDR1, CDR2 and CDR3 domains having the sequences set forth in SEQ ID NOs:4, 5 and 6, respectively. In another embodiment, the antibody comprises VH and VL regions having the amino acid sequences set forth in SEQ ID NO:12 and SEQ ID NO:8, respectively.

The exact boundaries of CDRs are defined differently according to different methods. In some embodiments, the positions of the CDRs or framework regions within a light or heavy chain variable domain are as defined by Kabat et al. [(1991) "Sequences of Proteins of Immunological Interest." NIH Publication No. 91-3242, U.S. Department of Health and Human Services, Bethesda, MD]. In such cases, the CDRs can be referred to as "Kabat CDRs" (*e.g.,* "Kabat LCDR2" or "Kabat HCDR1"). In some embodiments, the positions of the CDRs of a light or heavy chain variable region are as defined by Chothia et al. (Nature, 342:877-83, 1989). Accordingly, these regions can be referred to as "Chothia CDRs" (*e.g.,* "Chothia LCDR2" or "Chothia HCDR3"). In some embodiments, the positions of the CDRs of the light and heavy chain variable regions can be defined by a Kabat-Chothia combined definition. In such embodiments, these regions can be referred to as "combined Kabat-Chothia CDRs." Thomas, C. et al. (Mol. Immunol., 33:1389-401, 1996) exemplifies the identification of CDR boundaries according to Kabat and Chothia numbering schemes.

Another exemplary anti-C5 antibody is the 7086 antibody described in US Patent Nos. 8,241,628 and 8,883,158. In one embodiment, the antibody comprises the heavy and light chain CDRs or variable regions of the 7086 antibody (*see* US Patent Nos. 8,241,628 and 8,883,158). In another embodiment, the antibody, or antigen binding fragment thereof, comprises heavy chain CDR1, CDR2 and CDR3 domains having the sequences set forth in SEQ ID NOs:21, 22 and 23, respectively, and light chain CDR1, CDR2 and CDR3 domains having the sequences set forth in SEQ ID NOs:24, 25 and 26, respectively. In another embodiment, the antibody, or antigen binding fragment thereof, comprises the VH region of the 7086 antibody having the sequence set forth in SEQ ID NO:27, and the VL region of the 7086 antibody having the sequence set forth in SEQ ID NO:28.

Another exemplary anti-C5 antibody is the 8110 antibody also described in US Patent Nos. 8,241,628 and 8,883,158. In one embodiment, the antibody comprises the heavy and light chain CDRs or variable regions of the 8110 antibody. In another embodiment, the antibody, or antigen binding fragment thereof, comprises heavy chain CDR1, CDR2 and CDR3 domains having the sequences set forth in SEQ ID NOs:29, 30 and 31, respectively, and light chain CDR1, CDR2 and CDR3 domains having the sequences set forth in SEQ ID NOs:32, 33 and 34, respectively. In another embodiment, the antibody comprises the VH region of the 8110 antibody having the sequence set forth in SEQ ID NO:35, and the VL region of the 8110 antibody having the sequence set forth in SEQ ID NO:36.

Another exemplary anti-C5 antibody is the 305LO5 antibody described in US Patent No. 9,765,135. In one embodiment, the antibody comprises the heavy and light chain CDRs or variable regions of the 305LO5 antibody. In another embodiment, the antibody, or antigen binding fragment thereof, comprises heavy chain CDR1, CDR2 and CDR3 domains having the sequences set forth in SEQ ID NOs:37, 38 and 39, respectively, and light chain CDR1, CDR2 and CDR3 domains having the sequences set forth in SEQ ID NOs:40, 41 and 42, respectively. In another embodiment, the antibody comprises the VH region of the 305LO5 antibody having the sequence set forth in SEQ ID NO:43, and the VL region of the 305LO5 antibody having the sequence set forth in SEQ ID NO:44.

Another exemplary anti-C5 antibody is the SKY59 antibody (Fukuzawa, T. et al., Sci. Rep., 7:1080, 2017). In one embodiment, the antibody comprises the heavy and light chain CDRs or variable regions of the SKY59 antibody. In another embodiment, the antibody, or antigen binding fragment thereof, comprises a heavy chain comprising SEQ ID NO:45 and a light chain comprising SEQ ID NO:46.

In some embodiments, the anti-C5 antibody comprises the heavy and light chain variable regions or heavy and light chains of the REGN3918 antibody (see US Patent No. 10,633,434). In some embodiments, the anti-C5 antibody, or antigen-binding fragment thereof, comprises a heavy chain variable region sequence set forth in SEQ ID NO: 47 and a light chain variable region comprising the sequence set forth in SEQ ID NO: 48. In some embodiments, the anti-C5 antibody, or antigen-binding fragment thereof, comprises a heavy chain sequence set forth in SEQ ID NO: 49 and a light chain sequence set forth in SEQ ID NO: 50.

In some embodiments, an anti-C5 antibody described herein comprises a heavy chain CDR1 comprising, or consisting of, the following amino acid sequence: GHIFSNYWIQ (SEQ ID NO:19). In some embodiments, an anti-C5 antibody described herein comprises a heavy chain CDR2 comprising, or consisting of, the following amino acid sequence: EILPGSGHTEYTENFKD (SEQ ID NO:18). In some embodiments, an anti-C5 antibody described herein comprises a heavy chain variable region comprising the following amino acid sequence:




 (SEQ ID NO:12).

In some embodiments, an anti-C5 antibody described herein comprises a light chain variable region comprising the following amino acid sequence:



 (SEQ ID NO:8).

An anti-C5 antibody described herein can, in some embodiments, comprise a variant human Fc constant region that binds to human neonatal Fc receptor (FcRn) with greater affinity than that of the native human Fc constant region from which the variant human Fc constant region was derived. The Fc constant region can, for example, comprise one or more (e.g., two, three, four, five, six, seven, or eight or more) amino acid substitutions relative to the native human Fc constant region from which the variant human Fc constant region was derived. The substitutions can increase the binding affinity of an IgG antibody containing the variant Fc constant region to FcRn at pH 6.0, while maintaining the pH dependence of the interaction. Methods for testing whether one or more substitutions in the Fc constant region of an antibody increase the affinity of the Fc constant region for FcRn at pH 6.0 (while maintaining pH dependence of the interaction) are known in the art and exemplified in the working examples. See, e.g., WO2015134894 and US Patent No.9,079949 the disclosures of each of which are incorporated herein by reference in their entirety.

Substitutions that enhance the binding affinity of an antibody Fc constant region for FcRn are known in the art and include, e.g., (1) the M252Y/S254T/T256E triple substitution (Dall'Acqua, W. et al., J. Biol. Chem., 281:23514-24, 2006); (2) the M428L or T250Q/M428L substitutions (Hinton, P. et al., J. Biol. Chem., 279:6213-6, 2004; Hinton, P. et al., J. Immunol., 176:346-56, 2006); and (3) the N434A or T307/E380A/N434A substitutions (Petkova, S. et al., Int. Immunol., 18:1759-69, 2006). The additional substitution pairings: P257I/Q3111, P257I/N434H and D376V/N434H (Datta-Mannan, A. et al., J. Biol. Chem., 282:1709-17, 2007), the disclosures of each of which are incorporated herein by reference in their entirety.

In some embodiments, the variant constant region has a substitution at EU amino acid posaition 255 for valine. In some embodiments, the variant constant region has a substitution at EU amino acid position 309 for asparagine. In some embodiments, the variant constant region has a substitution at EU amino acid position 312 for isoleucine. In some embodiments, the variant constant region has a substitution at EU amino acid position 386.

In some embodiments, the variant Fc constant region comprises no more than 30 (*e*.*g*., no more than 29, 28, 27, 26, 25, 24, 23, 22, 21, 20, 19, 18, 17, 16, 15, 14, 13, 12, 11, 10, 9, 8, 7, 6, 5, 4, 3 or 2) amino acid substitutions, insertions, or deletions relative to the native constant region from which it was derived. In some embodiments, the variant Fc constant region comprises one or more amino acid substitutions selected from the group consisting of: M252Y, S254T, T256E, N434S, M428L, V259I, T250I and V308F. In some embodiments, the variant human Fc constant region comprises a methionine at position 428 and an asparagine at position 434 of a native human IgG Fc constant region, each in EU numbering. In some embodiments, the variant Fc constant region comprises a 428L/434S double substitution as described in, *e.g.,* U.S. Patent No. 8,088,376.

In some embodiments the precise location of these mutations may be shifted from the native human Fc constant region position due to antibody engineering. For example, the 428L/434S double substitution when used in a IgG2/4 chimeric Fc may correspond to 429L and 435S as in the M429L and N435S variants found in ravulizumab and described in US Patent Number 9,079,949 the disclosure of which is incorporated herein by reference in its entirety.

In some embodiments, the variant constant region comprises a substitution at amino acid position 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434 or 436 (EU numbering) relative to the native human Fc constant region. In some embodiments, the substitution is selected from the group consisting of: methionine for glycine at position 237; alanine for proline at position 238; lysine for serine at position 239; isoleucine for lysine at position 248; alanine, phenylalanine, isoleucine, methionine, glutamine, serine, valine, tryptophan, or tyrosine for threonine at position 250; phenylalanine, tryptophan, or tyrosine for methionine at position 252; threonine for serine at position 254; glutamic acid for arginine at position 255; aspartic acid, glutamic acid, or glutamine for threonine at position 256; alanine, glycine, isoleucine, leucine, methionine, asparagine, serine, threonine, or valine for proline at position 257; histidine for glutamic acid at position 258; alanine for aspartic acid at position 265; phenylalanine for aspartic acid at position 270; alanine, or glutamic acid for asparagine at position 286; histidine for threonine at position 289; alanine for asparagine at position 297; glycine for serine at position 298; alanine for valine at position 303; alanine for valine at position 305; alanine, aspartic acid, phenylalanine, glycine, histidine, isoleucine, lysine, leucine, methionine, asparagine, proline, glutamine, arginine, serine, valine, tryptophan, or tyrosine for threonine at position 307; alanine, phenylalanine, isoleucine, leucine, methionine, proline, glutamine, or threonine for valine at position 308; alanine, aspartic acid, glutamic acid, proline, or arginine for leucine or valine at position 309; alanine, histidine, or isoleucine for glutamine at position 311; alanine or histidine for aspartic acid at position 312;lysine or arginine for leucine at position 314; alanine or histidine for asparagine at position 315; alanine for lysine at position 317; glycine for asparagine at position 325; valine for isoleucine at position 332; leucine for lysine at position 334; histidine for lysine at position 360; alanine for aspartic acid at position 376; alanine for glutamic acid at position 380; alanine for glutamic acid at position 382; alanine for asparagine or serine at position 384; aspartic acid or histidine for glycine at position 385; proline for glutamine at position 386; glutamic acid for proline at position 387; alanine or serine for asparagine at position 389; alanine for serine at position 424; alanine, aspartic acid, phenylalanine, glycine, histidine, isoleucine, lysine, leucine, asparagine, proline, glutamine, serine, threonine, valine, tryptophan, or tyrosine for methionine at position 428; lysine for histidine at position 433; alanine, phenylalanine, histidine, serine, tryptophan, or tyrosine for asparagine at position 434; and histidine for tyrosine or phenylalanine at position 436, all in EU numbering.

Suitable anti-C5 antibodies for use in the methods described herein, in some embodiments, comprise a heavy chain polypeptide comprising the amino acid sequence set forth in SEQ ID NO: 14 and/or a light chain polypeptide comprising the amino acid sequence set forth in SEQ ID NO:11. Alternatively, the anti-C5 antibodies for use in the methods described herein, in some embodiments, comprise a heavy chain polypeptide comprising the amino acid sequence set forth in SEQ ID NO:20 and/or a light chain polypeptide comprising the amino acid sequence set forth in SEQ ID NO: 11.

In one embodiment, the antibody binds to C5 at pH 7.4 and 25C (and, otherwise, under physiologic conditions) with an affinity dissociation constant (K_{D}) that is at least 0.1 (*e.g.,* at least 0.15, 0.175, 0.2, 0.25, 0.275, 0.3, 0.325, 0.35, 0.375, 0.4, 0.425, 0.45, 0.475, 0.5, 0.525, 0.55, 0.575, 0.6, 0.625, 0.65, 0.675, 0.7, 0.725, 0.75, 0.775, 0.8, 0.825, 0.85, 0.875, 0.9, 0.925, 0.95, or 0.975) nM. In some embodiments, the K_{D} of the anti-C5 antibody, or antigen binding fragment thereof, is no greater than 1 (*e*.*g*., no greater than 0.9, 0.8, 0.7, 0.6, 0.5, 0.4, 0.3, or 0.2) nM.

In other embodiments, the [(K_{D} of the antibody for C5 at pH 6.0 at 25C)/(K_{D} of the antibody for C5 at pH 7.4 at 25C)] is greater than 21 (*e*.*g*., greater than 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 600, 700, 800, 900, 1000, 1500, 2000, 2500, 3000, 3500, 4000, 4500, 5000, 5500, 6000, 6500, 7000, 7500 or 8000).

Methods for determining whether an antibody binds to a protein antigen and/or the affinity for an antibody to a protein antigen are known in the art. The binding of an antibody to a protein antigen, for example, can be detected and/or quantified using a variety of techniques such as, but not limited to, Western blot, dot blot, surface plasmon resonance (SPR) detection (e.g., BIAcore system; Pharmacia Biosensor AB, Uppsala, Sweden and Piscataway, N.J.), or enzyme-linked immunosorbent assay (ELISA; Benny K. C. Lo (2004) "Antibody Engineering: Methods and Protocols," Humana Press (ISBN: 1588290921); Johne, B. et al., J. Immunol. Meth., 160: 191-8, 1993; Jönsson, U. et al., Ann. Biol. Clin., 51:19-26, 1993; Jönsson, U. et al., Biotechniques, 11:620-7, 1991). In addition, methods for measuring the affinity (*e.g.,* dissociation and association constants) are set forth in the working examples.

As used herein, the term "kₐ" refers to the rate constant for association of an antibody to an antigen. The term "k_{d}" refers to the rate constant for dissociation of an antibody from the antibody/antigen complex. And the term "K_{D}" refers to the equilibrium dissociation constant of an antibody-antigen interaction. The equilibrium dissociation constant is deduced from the ratio of the kinetic rate constants, K_{D} = kₐ/k_{d}. Such determinations can be measured, for example, at 25C or 37C (see the working examples). The kinetics of antibody binding to human C5 can be determined, for example, at pH 8.0, 7.4, 7.0, 6.5 and 6.0 via SPR on a BIAcore 3000 instrument using an anti-Fc capture method to immobilize the antibody.

In one embodiment, the anti-C5 antibody, or antigen binding fragment thereof, blocks the cleavage of C5 into C5a and C5b. Through this blocking effect, for example, the pro-inflammatory effects of C5a and the generation of the C5b-9 membrane attack complex (MAC) at the surface of a cell are inhibited.

Methods for determining whether a particular antibody described herein inhibits C5 cleavage are known in the art. Inhibition of human complement component C5 can reduce the cell-lysing ability of complement in a subject's body fluids. Such reductions of the cell-lysing ability of complement present in the body fluid(s) can be measured by methods known in the art such as, for example, by a conventional hemolytic assay such as the hemolysis assay (Kabat and Mayer (eds.), "Experimental Immunochemistry, 2nd Edition," 135-240, Springfield, IL, CC Thomas (1961), pages 135-139), or a conventional variation of that assay such as the chicken erythrocyte hemolysis method (Hillmen, P. et al., N. Engl. J. Med., 350:552-9, 2004). Methods for determining whether a candidate compound inhibits the cleavage of human C5 into forms C5a and C5b are known in the art (Evans, M. et al., Mol. Immunol., 32:1183-95, 1995). The concentration and/or physiologic activity of C5a and C5b in a body fluid can be measured, for example, by methods known in the art. For C5b, hemolytic assays or assays for soluble C5b-9 as discussed herein can be used. Other assays known in the art can also be used. Using assays of these or other suitable types, candidate agents capable of inhibiting human complement component C5 can be screened.

Immunological techniques such as, but not limited to, ELISA can be used to measure the protein concentration of C5 and/or its split products to determine the ability of an anti-C5 antibody, or antigen binding fragment thereof, to inhibit conversion of C5 into biologically active products. In some embodiments, C5a generation is measured. In some embodiments, C5b-9 neoepitope-specific antibodies are used to detect MAC formation.

Hemolytic assays can be used to determine the inhibitory activity of an anti-C5 antibody, or antigen binding fragment thereof, on complement activation. To determine the effect of an anti-C5 antibody, or antigen binding fragment thereof, on classical complement pathway-mediated hemolysis in a serum test solution *in vitro,* for example, sheep erythrocytes coated with hemolysin or chicken erythrocytes sensitized with anti-chicken erythrocyte antibody are used as target cells. The percentage of lysis is normalized by considering 100% lysis equal to the lysis occurring in the absence of the inhibitor. In some embodiments, the classical complement pathway is activated by a human IgM antibody, for example, as utilized in the Wieslab^{®} Classical Pathway Complement Kit (Wieslab^{®} COMPL CP310, Euro-Diagnostica, Sweden). Briefly, the test serum is incubated with an anti-C5 antibody, or antigen binding fragment thereof, in the presence of a human IgM antibody. The amount of C5b-9 that is generated is measured by contacting the mixture with an enzyme conjugated anti-C5b-9 antibody and a fluorogenic substrate and measuring the absorbance at the appropriate wavelength. As a control, the test serum is incubated in the absence of the anti-C5 antibody, or antigen binding fragment thereof. In some embodiments, the test serum is a CS-deficient serum reconstituted with a C5 polypeptide.

To determine the effect of an anti-C5 antibody, or antigen binding fragment thereof, on alternative pathway-mediated hemolysis, unsensitized rabbit or guinea pig erythrocytes can be used as the target cells. In some embodiments, the serum test solution is a C5-deficient serum reconstituted with a C5 polypeptide. The percentage of lysis is normalized by considering 100% lysis equal to the lysis occurring in the absence of the inhibitor. In some embodiments, the alternative complement pathway is activated by lipopolysaccharide molecules, for example, as utilized in the Wieslab^{®} Alternative Pathway Complement Kit (Wieslab^{®} COMPL AP330, Euro-Diagnostica, Sweden). Briefly, the test serum is incubated with an anti-C5 antibody, or antigen binding fragment thereof, in the presence of lipopolysaccharide. The amount of C5b-9 that is generated is measured by contacting the mixture with an enzyme conjugated anti-C5b-9 antibody and a fluorogenic substrate and measuring the fluorescence at the appropriate wavelength. As a control, the test serum is incubated in the absence of the anti-C5 antibody, or antigen binding fragment thereof.

In some embodiments, C5 activity, or inhibition thereof, is quantified using a CH50eq assay. The CH50eq assay is a method for measuring the total classical complement activity in serum. This test is a lytic assay, which uses antibody-sensitized erythrocytes as the activator of the classical complement pathway and various dilutions of the test serum to determine the amount required to give 50% lysis (CH50). The percent hemolysis can be determined, for example, using a spectrophotometer. The CH50eq assay provides an indirect measure of terminal complement complex (TCC) formation, since the TCC themselves are directly responsible for the hemolysis that is measured. The assay is known and commonly practiced by those of skill in the art. Briefly, to activate the classical complement pathway, undiluted serum samples (*e*.*g*., reconstituted human serum samples) are added to microassay wells containing the antibody-sensitized erythrocytes to thereby generate TCC. Next, the activated sera are diluted in microassay wells, which are coated with a capture reagent (*e.g.,* an antibody that binds to one or more components of the TCC). The TCC present in the activated samples bind to the monoclonal antibodies coating the surface of the microassay wells. The wells are washed and to each well is added a detection reagent that is detectably labeled and recognizes the bound TCC. The detectable label can be, *e.g.,* a fluorescent label or an enzymatic label. The assay results are expressed in CH50 unit equivalents per milliliter (CH50 U Eq/mL).

Inhibition, *e*.*g*., as it pertains to terminal complement activity, includes at least a 5 (*e.g.,* at least a 6, 7, 8, 9, 10, 15, 20, 25, 30, 35, 40, 45, 50, 55 or 60) % decrease in the activity of terminal complement in, *e*.*g*., a hemolytic assay or CH50eq assay as compared to the effect of a control antibody (or antigen-binding fragment thereof) under similar conditions and at an equimolar concentration. Substantial inhibition, as used herein, refers to inhibition of a given activity (e.g., terminal complement activity) of at least 40 (*e.g.,* at least 45, 50, 55, 60, 65, 70, 75, 80, 85, 90, or 95 or greater) %. In some embodiments, an anti-C5 antibody described herein contains one or more amino acid substitutions relative to the CDRs of eculizumab (*i.e.,* SEQ ID NOs:1-6), yet retains at least 30 (*e.g.,* at least 31, 32, 33, 34, 35, 36, 37, 38, 39, 40, 41, 42, 43, 44, 45, 46, 47, 48, 49, 50, 55, 60, 65, 70, 75, 80, 85, 90 or 95) % of the complement inhibitory activity of eculizumab in a hemolytic assay or CH50eq assay.

An anti-C5 antibody described herein has a serum half-life in humans that is at least 20 (*e.g.,* at least 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55) days. In another embodiment, the anti-C5 antibody described herein has a serum half-life in humans that is at least 40 days. In another embodiment, the anti-C5 antibody described herein has a serum half-life in humans that is approximately 43 days. In another embodiment, the anti-C5 antibody described herein has a serum half-life in humans that is between 39-48 days. Methods for measuring the serum half-life of an antibody are known in the art. In some embodiments, an anti-C5 antibody, or antigen binding fragment thereof, described herein has a serum half-life that is at least 20 (*e.g.,* at least 30, 35, 40, 45, 50, 55, 60, 65, 70, 75, 80, 85, 90, 95, 100, 125, 150, 175, 200, 250, 300, 400 or 500) % greater than the serum half-life of eculizumab, *e.g.,* as measured in one of the mouse model systems described in the working examples (*e.g.,* the C5-deficient/NOD/scid mouse or hFcRn transgenic mouse model system).

In one embodiment, the antibody competes for binding with, and/or binds to the same epitope on C5 as an antibody described herein. The term "binds to the same epitope" with reference to two or more antibodies means that the antibodies bind to the same segment of amino acid residues, as determined by a given method. Techniques for determining whether antibodies bind to the same epitope on C5 with an antibody described herein include, for example, epitope mapping methods, such as, x-ray analyses of crystals of antigen: antibody complexes, and hydrogen/deuterium exchange mass spectrometry (HDX-MS). Other methods monitor the binding of the antibody to peptide antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same VH and VL or the same CDR1, CDR2 and CDR3 sequences are expected to bind to the same epitope.

Antibodies that "compete with another antibody for binding to a target" refer to antibodies that inhibit (partially or completely) the binding of the other antibody to the target. Whether two antibodies compete with each other for binding to a target, *i*.*e*., whether and to what extent one antibody inhibits the binding of the other antibody to a target, may be determined using known competition experiments. In certain embodiments, an antibody competes with, and inhibits binding of another antibody to a target by at least 10%, 20%, 30%, 40%, 50%, 60%, 70%, 80%, 90% or 100%. The level of inhibition or competition may be different depending on which antibody is the "blocking antibody" (*i*.*e*., the antibody that is incubated first with the target). Competing antibodies can bind to, for example, the same epitope, an overlapping epitope or to adjacent epitopes (*e.g.,* as evidenced by steric hindrance).

Anti-C5 antibodies, or antigen-binding fragments thereof described herein, used in the methods described herein can be generated using a variety of art-recognized techniques. Monoclonal antibodies can be obtained by various techniques familiar to those skilled in the art. Briefly, spleen cells from an animal immunized with a desired antigen are immortalized, commonly by fusion with a myeloma cell (Köhler, G. & Milstein, C., Eur. J. Immunol., 6:511-9, 1976)). Methods of immortalization include transformation with Epstein Barr Virus, oncogenes, or retroviruses or other methods known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences that encode a monoclonal antibody or a binding fragment thereof by screening a DNA library from human B cells (Huse, W. et al., Science, 246:1275-81, 1989).

### III. Compositions

Also provided herein are compositions comprising an anti-C5 antibody or antigen binding fragment thereof. In one embodiment, the composition comprises an anti-C5 antibody comprising the CDR1, CDR2 and CDR3 domains in a heavy chain variable region having the sequence set forth in SEQ ID NO:12, and the CDR1, CDR2 and CDR3 domains in a light chain variable region having the sequence set forth in SEQ ID NO:8. In another embodiment, the anti-C5 antibody comprises heavy and light chains having the sequences shown in SEQ ID NOs:14 and 11, respectively. In another embodiment, the anti-C5 antibody comprises heavy and light chains having the sequences shown in SEQ ID NOs:20 and 11, respectively.

The compositions can be formulated as a pharmaceutical solution, *e.g.,* for administration to a subject for the treatment or prevention of a complement-associated disorder. The pharmaceutical compositions generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, *e.g.,* an acid addition salt or a base addition salt, sugars, carbohydrates, polyols and/or tonicity modifiers.

The compositions can be formulated according to standard methods. Pharmaceutical formulation is an established art (see, for example, Gennaro (2000) "Remington: The Science and Practice of Pharmacy," 20th Edition, Lippincott, Williams & Wilkins (ISBN: 0683306472); Ansel et al. (1999) "Pharmaceutical Dosage Forms and Drug Delivery Systems," 7th Edition, Lippincott Williams & Wilkins Publishers (ISBN: 0683305727); and Kibbe (2000) "Handbook of Pharmaceutical Excipients American Pharmaceutical Association," 3rd Edition (ISBN: 091733096X)). In some embodiments, a composition can be formulated, for example, as a buffered solution at a suitable concentration and suitable for storage at 2-8C (*e*.*g*., 4C). In some embodiments, a composition can be formulated for storage at a temperature below 0C (*e.g.,* -20C or -80C). In some embodiments, the composition can be formulated for storage for up to 2 years (*e.g.,* 1 month, 2 months, 3 months, 4 months, 5 months, 6 months, 7 months, 8 months, 9 months, 10 months, 11 months, 1 year, 1½years or 2 years) at 2-8C (e.g., 4C). Thus, in some embodiments, the compositions described herein are stable in storage for at least 1 year at 2-8C (*e.g.,* 4C).

The pharmaceutical compositions can be in a variety of forms. These forms include, *e.g.,* liquid, semi-solid and solid dosage forms, such as liquid solutions (*e.g.,* injectable and infusible solutions), dispersions or suspensions, tablets, pills, powders, liposomes and suppositories. The preferred form depends, in part, on the intended mode of administration and therapeutic application. Compositions containing a composition intended for systemic or local delivery, for example, can be in the form of injectable or infusible solutions. Accordingly, the compositions can be formulated for administration by a parenteral mode (e.g., intravenous, subcutaneous, intraperitoneal, or intramuscular injection). "Parenteral administration," "administered parenterally" and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid and intrasternal injection and infusion.

### IV. Methods

Provided herein are methods for treating PNH in a human pediatric patient, comprising administering to the patient an anti-C5 antibody, or antigen binding fragment thereof, wherein the anti-C5 antibody or antigen binding fragment thereof is administered (or is for administration) according to a particular clinical dosage regimen (*e.g.,* at a particular dose amount and according to a specific dosing schedule).

In one embodiment, the dose of the anti-C5 antibody, or antigen binding fragment thereof, is based on the weight of the patient. In one embodiment, for example, 300 mg of the anti-C5 antibody or antigen binding fragment thereof is administered to a patient weighing ≥ 5 to < 10 kg. In another embodiment, 600 mg of the anti-C5 antibody or antigen binding fragment thereof is administered to a patient weighing ≥ 10 to < 20 kg. In another embodiment, 900 mg or 2100 mg of the anti-C5 antibody or antigen binding fragment thereof is administered to a patient weighing > 20 to < 30 kg. In another embodiment, 1200 mg or 2700 mg of the anti-C5 antibody or antigen binding fragment thereof is administered to a patient weighing ≥ 30 to < 40 kg. In another embodiment, 2400 mg or 3000 mg of the anti-C5 antibody or antigen binding fragment thereof is administered to a patient weighing ≥ 40 to < 60 kg. In another embodiment, 2700 mg or 3300 mg of the anti-C5 antibody or antigen binding fragment thereof is administered to a patient weighing ≥ 60 to < 100 kg. In another embodiment, 3000 mg or 3600 mg of the anti-C5 antibody or antigen binding fragment thereof is administered to a patient weighing ≥ 100 kg. In certain embodiments, dosage regimens are adjusted to provide the optimum desired response (e.g., an effective response).

In another embodiment, the anti-C5 antibody or antigen binding fragment thereof is administered for one or more administration cycles. In one embodiment, the treatment (*e*.*g*., administration cycle) is 26 weeks. In one embodiment, the anti-C5 antibody or antigen binding fragment thereof is administered once on Day 1 (*e*.*g*., of the administration cycle), once on Day 15 (*e.g.,* of the administration cycle), and every four weeks thereafter. In another embodiment, the anti-C5 antibody or antigen binding fragment thereof is administered once on Day 1 (*e.g.,* of the administration cycle), once on Day 15 (*e.g.,* of the administration cycle), and every eight weeks thereafter. In another embodiment, the anti-C5 antibody or antigen binding fragment thereof is administered every four or eight weeks after treatment (*e*.*g*., an administration cycle) for an extension period up to two years (*e.g.,* at a dose of 300 mg, 600 mg, 900 mg, 1200 mg, 2100 mg, 2400 mg, 2700 mg, 3000 mg, 3300 mg or 3600 mg).

In another embodiment, a method of treating a human patient with PNH is provided, the method comprising administering to the patient (e.g., during an administration cycle) an effective amount of an anti-C5 antibody or antigen binding fragment thereof, comprising CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively, wherein the anti-C5 antibody or antigen binding fragment thereof is administered: (a) once on Day 1 at a dose of 600 mg to a patient weighing > 5 to < 10 kg, 600 mg to a patient weighing > 10 to < 20 kg, 900 mg to a patient weighing > 20 to < 30 kg, 1200 mg to a patient weighing > 30 to < 40 kg, 2400 mg to a patient weighing > 40 to < 60 kg, 2700 mg to a patient weighing > 60 to < 100 kg, or 3000 mg to a patient weighing ≥ 100 kg; and (b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing > 30 to < 40 kg, 3000 mg to a patient weighing > 40 to < 60 kg, 3300 mg to a patient weighing > 60 to < 100 kg, or 3600 mg to a patient weighing > 100 kg.

In another embodiment, a method of treating a human patient with PNH is provided, the method comprising administering to the patient (*e.g.,* during an administration cycle) an effective amount of an anti-C5 antibody or antigen binding fragment thereof, comprising CDR1, CDR2 and CDR3 heavy chain sequences as set forth in SEQ ID NOs: 19, 18 and 3, respectively, and CDR1, CDR2 and CDR3 light chain sequences as set forth in SEQ ID NOs:4, 5 and 6, respectively, wherein the anti-C5 antibody or antigen binding fragment thereof is administered:
(a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing > 30 to < 40 kg, 2400 mg to a patient weighing > 40 to < 60 kg, 2700 mg to a patient weighing > 60 to < 100 kg, or 3000 mg to a patient weighing ≥ 100 kg; and (b) on Day 15 and every four weeks thereafter at a dose of 300 mg to a patient weighing > 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing > 40 to < 60 kg, 3300 mg to a patient weighing ≥ 60 to < 100 kg, or 3600 mg to a patient weighing > 100 kg, and wherein the treatment results in a reduction in LDH levels to within normal levels or to within 50% below what is considered the ULN level (*e*.*g*., within 105-333 IU/L (international units per liter), a serum trough concentration of the anti-C5 antibody, or antigen binding fragment thereof, of at least 175 µg/mL or greater, and/or a free C5 concentration of 0.5 µg/mL or less (*e.g.,* 0.4 µg/mL, 0.3 µg/mL, 0.2 µg/mL, or 0.1 µg/mL or less). In another embodiment, the treatment eliminates breakthrough hemolysis (BTH).

In another embodiment, a method of treating a human patient with PNH is provided, the method comprising administering to the patient (*e.g.,* during an administration cycle) an effective amount of an anti-C5 antibody or antigen binding fragment thereof comprising CDR1, CDR2 and CDR3 heavy chain sequences as set forth in SEQ ID NOs: 19, 18 and 3, respectively, CDR1, CDR2 and CDR3 light chain sequences as set forth in SEQ ID NOs:4, 5 and 6, respectively, and a variant human Fc constant region that binds to human neonatal Fc receptor (FcRn), wherein the variant human Fc CH3 constant region comprises Met429Leu and Asn435Ser substitutions at residues corresponding to methionine 428 and asparagine 434 of a native human IgG Fc constant region, each according to the EU numbering convention, wherein the anti-C5 antibody or antigen binding fragment thereof is administered: (a) once on Day 1 at a dose of 600 mg to a patient weighing > 5 to < 10 kg, 600 mg to a patient weighing ≥ 10 to < 20 kg, 900 mg to a patient weighing > 20 to < 30 kg, 1200 mg to a patient weighing ≥ 30 to < 40 kg, 2400 mg to a patient weighing > 40 to < 60 kg, 2700 mg to a patient weighing ≥ 60 to < 100 kg, or 3000 mg to a patient weighing ≥ 100 kg; and (b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In another embodiment, the anti-C5 antibody or antigen binding fragment thereof is administered to a patient weighing > 5 to < 10 kg: (a) once on Day 1 at a dose of 600 mg; and (b) on Day 15 and every four weeks thereafter at a dose of 300 mg.

In another embodiment, the anti-C5 antibody is administered to a patient weighing ≥ 10 to < 20 kg: (a) once on Day 1 at a dose of 600 mg; and (b) on Day 15 and every four weeks thereafter at a dose of 600 mg.

In another embodiment, the anti-C5 antibody is administered to a patient weighing ≥ 20 to < 30 kg: (a) once on Day 1 at a dose of 900 mg; and (b) on Day 15 and every eight weeks thereafter at a dose of 2100 mg.

In another embodiment, the anti-C5 antibody is administered to a patient weighing ≥ 30 to < 40 kg: (a) once on Day 1 at a dose of 1200 mg; and (b) on Day 15 and every eight weeks thereafter at a dose of 2700 mg.

In another embodiment, the anti-C5 antibody is administered to a patient weighing ≥ 40 to < 60 kg: (a) once on Day 1 at a dose of 2400 mg; and (b) on Day 15 and every eight weeks thereafter at a dose of 3000 mg.

In another embodiment, the anti-C5 antibody is administered to a patient weighing ≥ 60 to < 100 kg: (a) once on Day 1 at a dose of 2700 mg; and (b) on Day 15 and every eight weeks thereafter at a dose of 3300 mg.

In another embodiment, the anti-C5 antibody is administered to a patient weighing ≥ 100 kg: (a) once on Day 1 at a dose of 3000 mg; and (b) on Day 15 and every eight weeks thereafter at a dose of 3600 mg.

In one embodiment, the patient has not previously been treated with eculizumab. In another embodiment, the patient has previously been treated with eculizumab. In another embodiment, the patient has previously been treated with eculizumab and Day 1 (*e.g.,* of the administration cycle) is two weeks or more from the patient's last dose of eculizumab.

### V. Outcomes

Provided herein are methods for treating PNH in a patient comprising administering to the patient an anti-C5 antibody. Symptoms of PNH include, but are not limited to, pallor, fatigue (*e*.*g*., tiredness, difficultly performing daily activities, trouble concentrating, dizziness, weakness), pain (*e.g.,* stomach pain, leg pain or swelling, chest pain, back pain), dark-colored urine, shortness of breath, difficulty swallowing, yellowing of the skin and/or eyes, anemia, cytopenia, erectile dysfunction, blood clots, kidney disease, damage to organs, stroke or heart attack.

Patients treated according to the methods disclosed herein experience improvement in at least one sign of PNH. The treatment may produce at least one therapeutic effect selected from the group consisting of, for example, a reduction or cessation in pallor, fatigue, jaundice, anemia, cytopenia, abdominal pain, dyspnea, dysphagia, chest pain or erectile dysfunction.

In one embodiment, improvement is measured by terminal complement inhibition.

In another embodiment, lactate dehydrogenase (LDH) levels can be used to evaluate responsiveness to a therapy (*e.g.,* a reduction of hemolysis as assessed by lactate dehydrogenase (LDH) levels is indicative of an improvement in at least one sign of PNH). LDH is a marker of intravascular hemolysis (Hill, A. et al., Br. J. Haematol., 149:414-25, 2010; Hillmen, P. et al., N. Engl. J. Med., 350:552-9, 2004; Parker, C. et al., Blood, 106:3699-709, 2005). Red blood cells contain large amounts of LDH, and a correlation between cell-free hemoglobin and LDH concentration has been reported *in vitro* (Van Lente, F. et al., Clin. Chem., 27:1453-5, 1981) and *in vivo* (Kato, G. et al., Blood, 107:2279-85, 2006). The consequences of hemolysis are independent of anemia (Hill, A. et al., Haematologica, 93(s1):359 Abs.0903, 2008; Kanakura, Y. et al., Int. J. Hematol., 93:36-46, 2011). LDH concentration obtained at baseline and then serially throughout a treatment period, is an important measure of hemolysis. Baseline levels of cell-free plasma hemoglobin are highly elevated in patients with PNH with LDH ≥1.5-fold above the upper limit of normal (LDH ≥ 1.5 × ULN), with a significant correlation between LDH and cell-free plasma hemoglobin (Hillmen, P. et al., N. Engl. J. Med., 355: 1233-43, 2006). The normal LDH value range is 105-333 IU/L (international units per liter).

LDH levels can be measured using any suitable test or assay, such as those described by Ferri FF, ed. Ferri's Clinical Advisor 2014. Philadelphia: Pa: Elsevier Mosby; 2014: Section IV- Laboratory tests and interpretation of results. LDH concentration can be measured in various samples obtained from a patient, in particular, serum samples. As used herein, the term "sample" refers to biological material from a subject. Although serum LDH concentration is of interest, samples can be derived from other sources, including, for example, single cells, multiple cells, tissues, tumors, biological fluids, biological molecules or supernatants or extracts of any of the foregoing. Examples include tissue removed for biopsy, tissue removed during resection, blood, urine, lymph tissue, lymph fluid, cerebrospinal fluid, mucous and stool samples. The sample used can vary based on the assay format, the detection method and the nature of the tumors, tissues, cells or extracts to be assayed. Methods for preparing samples are known in the art and can be readily adapted to obtain a sample that is compatible with the method utilized.

In one embodiment, patients treated according to the disclosed methods experience reductions in LDH levels to normal levels or to within 10%, or within 20% above what is considered the normal level (e.g., within 105-333 IU/L). For example, patients treated according to the disclosed methods experience reductions in LDH levels to within normal levels or to within 10%, 11%. 12%,13%, 14%, 15%, 16%, 17%, 18%, 19%, 20% 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or within 50% below what is considered the ULN level (*e*.*g*., within 105-333 IU/L (international units per liter). In one embodiment, the patient's LDH levels are ≥ 1.5 fold above the ULN (LDH ≥ 1.5 × ULN) prior to initiating treatment.

In one embodiment, patients treated according to the disclosed methods experience an LDH percent change compared to baseline of 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53% 54%, 55%, 56%, 57%, 58%, 59%, or 60%.

In one embodiment, patients treated according to the disclosed methods maintain a serum trough concentration of the anti-C5 antibody, or antigen binding fragment thereof, of at least 150, 155, 160, 165, 170, 175, 180, 185, 190, 200, 205, 210, 215, 220, 225, 230, 240, 245, 250, 255, 260, 265, 270, 280, 290, 300, 305, 310, 315, 320, 325, 330, 335, 340, 345, 350, 355, 360, 365, 370, 375, 380, 385, 390, 395 or 400 µg/mL or greater. In one embodiment, patients treated according to the disclosed methods maintain a serum trough concentration of the anti-C5 antibody, or antigen binding fragment thereof, of at least 175 µg/mL or greater.

In one embodiment, patients treated according to the disclosed methods have a free C5 concentration of 0.5 µg/mL or less (*e.g.,* 0.4 µg/mL, 0.3 µg/mL, 0.2 µg/mL, or 0.1 µg/mL or less).

In one embodiment, patients treated according to the disclosed methods do not experience breakthrough hemolysis (BTH).

In another embodiment, the treatment produces a shift toward normal levels of a hemolysis-related hematologic biomarker selected from the group consisting of free hemoglobin, haptoglobin, reticulocyte count, PNH red blood cell (RBC) clone and D-dimer.

In another embodiment, the treatment produces a reduction in the need for blood transfusions compared to baseline.

In another embodiment, the treatment produces a reduction in major adverse vascular events (MAVEs; *e*.*g*., thrombophlebitis/deep vein thrombosis, pulmonary embolus, myocardial infarction, transient ischemic attack, unstable angina, renal vein thrombosis/renal artery thrombosis/glomerular thrombosis, renal infarction, acute peripheral vascular occlusion, mesenteric/visceral vein/arterial thrombosis or infarction, hepatic/portal vein thrombosis, cerebral arterial occlusion/cerebrovascular accident, cerebral venous occlusion, renal arterial thrombosis, or multi-infarct dementia).

In another embodiment, the treatment produces a shift toward normal levels of a chronic disease associated biomarker selected from the group consisting estimated glomerular filtration rate (eGFR) and spot urine: albumin: creatinine and plasma brain natriuretic peptide (BNP).

In another embodiment, the treatment produces a change from baseline in quality of life as assessed via the Functional Assessment of Chronic Illness Therapy (FACIT)-Fatigue Scale, version 4 and the European Organisation for Research and Treatment of Cancer, Quality of Life Questionnaire-Core 30 Scale compared to baseline.

### VI. Kits and Unit Dosage Forms

Also provided herein are kits that include a pharmaceutical composition containing an anti-C5 antibody or antigen binding fragment thereof, such as ravulizumab or BNJ421, and a pharmaceutically acceptable carrier, in a therapeutically effective amount adapted for use in the preceding methods. The kits optionally also can include instructions, *e.g.,* comprising administration schedules, to allow a practitioner (*e*.*g*., a physician, nurse, or patient) to administer the composition contained therein to administer the composition to a patient having PNH. The kit also can include a syringe.

Optionally, the kits include multiple packages of the single-dose pharmaceutical compositions each containing an effective amount of the anti-C5 antibody, or antigen binding fragment thereof, for a single administration in accordance with the methods provided above. Instruments or devices necessary for administering the pharmaceutical composition(s) also may be included in the kits. For instance, a kit may provide one or more pre-filled syringes containing an amount of the anti-C5 antibody or antigen binding fragment thereof.

In one embodiment, a kit for treating PNH in a human pediatric patient comprises: (a) a dose of an anti-C5 antibody or antigen binding fragment thereof, comprising CDR1, CDR2 and CDR3 domains of the heavy chain variable region having the sequence set forth in SEQ ID NO: 12, and CDR1, CDR2 and CDR3 domains of the light chain variable region having the sequence set forth in SEQ ID NO:8; and (b) instructions for using the anti-C5 antibody or antigen binding fragment thereof, according to any of the methods described herein.

In one embodiment, the kit comprises a dose of an anti-C5 antibody or antigen binding fragment thereof, wherein the anti-C5 antibody or antigen binding fragment thereof, is administered to a patient weighing ≥ 5 to < 10 kg: (a) once on Day 1 at a dose of 600 mg; and (b) on Day 15 and every four weeks thereafter at a dose of 300 mg.

In another embodiment, the kit comprises a dose of an anti-C5 antibody or antigen binding fragment thereof, wherein the anti-C5 antibody or antigen binding fragment thereof, is administered to a patient weighing > 10 to < 20 kg: (a) once on Day 1 at a dose of 600 mg; and (b) on Day 15 and every four weeks thereafter at a dose of 600 mg.

In another embodiment, the kit comprises a dose of an anti-C5 antibody or antigen binding fragment thereof, wherein the anti-C5 antibody, or antigen binding fragment thereof, is administered to a patient weighing ≥ 20 to < 30 kg: (a) once on Day 1 at a dose of 900 mg; and (b) on Day 15 and every eight weeks thereafter at a dose of 2100 mg.

In another embodiment, the kit comprises a dose of an anti-C5 antibody or antigen binding fragment thereof, wherein the anti-C5 antibody, or antigen binding fragment thereof, is administered to a patient weighing ≥ 30 to < 40 kg: (a) once on Day 1 at a dose of 1200 mg; and (b) on Day 15 and every eight weeks thereafter at a dose of 2700 mg.

In another embodiment, the kit comprises a dose of an anti-C5 antibody, or antigen binding fragment thereof, wherein the anti-C5 antibody, or antigen binding fragment thereof, is administered to a patient weighing > 40 to < 60 kg: (a) once on Day 1 at a dose of 2400 mg; and (b) on Day 15 and every eight weeks thereafter at a dose of 3000 mg.

In another embodiment, the kit comprises a dose of an anti-C5 antibody or antigen binding fragment thereof, wherein the anti-C5 antibody, or antigen binding fragment thereof, is administered to a patient weighing ≥ 60 to < 100 kg: (a) once on Day 1 at a dose of 2700 mg; and (b) on Day 15 and every eight weeks thereafter at a dose of 3300 mg.
In another embodiment, the kit comprises a dose of an anti-C5 antibody or antigen binding fragment thereof, wherein the anti-C5 antibody, or antigen binding fragment thereof, is administered to a patient weighing ≥ 100 kg: (a) once on Day 1 at a dose of 3000 mg; and (b) on Day 15 and every eight weeks thereafter at a dose of 3600 mg.

The following examples are merely illustrative and should not be construed as limiting the scope of this disclosure in any way as many variations and equivalents will become apparent to those skilled in the art upon reading the present disclosure. The contents of all references, Genbank entries, patents and published patent applications cited throughout this application are expressly incorporated herein by reference.

### EXAMPLES

### EXAMPLE 1: A Phase 3, Open-Label Study of Ravulizumab in Children and Adolescents with Paroxysmal Nocturnal Hemoglobinuria (PNH)

In this Phase 3, open-label study, the pharmacokinetic(s) (PK), pharmacodynamics (PD), efficacy, and safety of ravulizumab are assessed in pediatric patients with PNH.

### 1. Objectives

The primary objective of the study is to assess the PK, PD, safety and efficacy of ravulizimab in pediatric patients with PNH.

### 2. Endpoints

The primary endpoints are PK/PD parameters (trough and peak) at Baseline and Weeks 2, 10, 18, and 26. PK parameters include maximum serum concentration (Cₘₐₓ), trough serum concentration (measured at end of dosing interval at steady state; C_{trough}), and accumulation ratio. PD parameters include measuring the change in free C5 concentrations and in chicken red blood cell (cRBC) hemolytic activity over time.

Secondary endpoints include assessment of the following parameters:
Percentage change in LDH from baseline to Day 183 (Week 26);
Transfusion avoidance (TA), defined as the proportion of patients who remain transfusion-free and do not require a transfusion through Day 183 (Week 26);
Change in quality of life (QoL), as measured by Pediatric Functional Assessment of Chronic Therapy (FACIT) Fatigue questionnaire (patients ≥5 years of age), from baseline to Day 183 (Week 26);
Proportion of patients with stabilized hemoglobin, defined as avoidance of a ≥2 g/dL decrease in hemoglobin level from baseline in the absence of transfusion through Day 183 (Week 26);
Percentage change in free hemoglobin from baseline to Day 183 (Week 26);
Proportion of patients with breakthrough hemolysis, defined as at least one new or worsening symptom or sign of intravascular hemolysis (fatigue, hemoglobinuria, abdominal pain, shortness of breath [dyspnea], anemia, major adverse vascular event [MAVE, including thrombosis], dysphagia, or erectile dysfunction) in the presence of elevated LDH as follows: for patients who enter the study naive to complement inhibitor treatment, elevated LDH ≥2 × the upper limit of normal (ULN) after prior LDH reduction to <1.5 × ULN on therapy; for patients who enter the study stabilized on eculizumab treatment, elevated LDH ≥2 × ULN.

With respect to safety endpoints, the safety and tolerability of ravulizumab is evaluated from baseline to Week 26 and throughout the extension period by physical examinations, vital signs, physical growth (height, weight, and head circumference [the latter only in patients who are ≤ 3 years of age]), electrocardiograms (ECGs), laboratory assessments, and incidence of adverse events (AEs) and serious adverse events (SAEs). The proportion of patients who develop antidrug antibodies (ADAs) is also assessed.

### 3. Study Design

This is a Phase 3, open-label, single-arm multicenter study to evaluate the PK7PD, safety, and efficacy of ravulizumab administered by intravenous (IV) infusion to pediatric patients (< 18 years of age) with PNH. The study consists of a 4-week Screening Period, a 26-week Primary Evaluation Period and an Extension Period.

Consenting patients are screened for study eligibility up to 4 weeks prior to Day 1. Patients who satisfy all of the inclusion criteria and all of the exclusion criteria are enrolled into the Primary Evaluation Period and receive a weight-based loading dose of ravulizumab on Day 1, followed by weight-based maintenance treatment with ravulizumab on Day 15 and once every 8 weeks (q8w) thereafter for patients weighing ≥20 kg, or once every 4 weeks (q4w) for patients weighing <20 kg, for a total of 26 weeks of treatment. For patients entering the study on eculizumab therapy, Day 1 of study treatment occurs 2 weeks from the patient's last dose of eculizumab.

An interim analysis of data, including ravulizumab PK and free C5 levels, is conducted after 4 patients weighing ≥5 kg to <40 kg have completed dosing through Day 71. Enrollment of patients proceeds without interruption while the analysis is ongoing. The accrued safety and PK/PD data is assessed to ensure that ravulizumab treatment is well tolerated and is providing adequate complement inhibition. In addition, an independent Data Monitoring Committee (DMC) reviews safety data from the study on a regular basis.

After completion of all assessments on Day 183, all patients enter an Extension Period and continue to receive ravulizumab according to the appropriate weight-based regimen. The Extension Period continues until the product is registered or approved (in accordance with country-specific regulations) or for up to 2 years, whichever occurs first. The end of trial is defined as the last patient's last visit in the Extension Period.

### 4. Schedule of Assessments

The Schedule of Assessments is set forth in Table 1 for the Screening and Primary Evaluation Period and in Table 2 for the Extension Period.

Additional (unscheduled) visits outside the specified visits are permitted at the discretion of the Investigator. Procedures, tests, and assessments are performed at the discretion of the Investigator. All tests, procedures, or assessments performed at the Unscheduled Visits are recorded on the electronic case report forms (eCRFs).

Additionally, if a suspected event of breakthrough hemolysis occurs, LDH, PK and PD parameters are analyzed at the central laboratory. If the suspected event of breakthrough does not occur at a scheduled visit, an unscheduled visit occurs for evaluation of the patient and collection of the required LDH, PK and PD parameters. For purposes of defining breakthrough hemolysis, assessment of LDH is based on a central laboratory value.

**Table 1: Schedule of Study Visits and Assessments: Screening Through End of Primary Evaluation Period**

| **Period** | **Screening** | **Initial Evaluation Period** | | | | | | | |
|---|---|---|---|---|---|---|---|---|---|
| **Study Day** | **-28 to -1** | **1** | **15** | **43** | **71** | **99** | **127** | **155** | **183^{a}/ET** |
| **Window (day)** | **N/A** | | **±3** | **±3** | **±3** | **±5** | **±5** | **±5** | **±2** |
| Informed consent | X | | | | | | | | |
| Confirmation or administration of meningococcal vaccination^{b} | X | X | | | | | | | |
| Confirmation of H influenza type B and S pneumoniae vaccination (per local/national guidelines) | X | | | | | | | | |
| Medical history and demographics | X | | | | | | | | |
| PNH clone size^{c} | X | X | | | X | | | | X |
| Head circumference (patients ≤ 3 years of age only) | X | X | X | X | X | X | X | X | X |
| Height and weight^{d} | X | X | X | X | X | X | X | X | X |
| Pregnancy test^{e} | X | X | X | | X | | X | | X |
| Record transfusions (during and between visits) | X | X | X | X | X | X | X | X | X |
| PNH svmptomatology^{f} | X | X | X | X | X | X | X | X | X |
| Pediatric FACIT-Fatigue questionnaire^{g} | | X | X | | X | | X | | X |
| Physical examination | X | | | | | | | | X |
| Abbreviated physical examination^{h} | | X | X | X | X | X | X | X | |
| Vital signsⁱ | X | X | X | X | X | X | X | X | X |
| Safety 12-Lead ECG^{j} | X | | | | X | | | | X |
| Chemistry including LDH^{k} | X¹ | X | X | X | X | X | X | X | X |
| Hematology including free hemoglobin and coagulation^{k} | X | X | X | X | X | X | X | X | X |
| Urinalysis and urine chemistry^{k} | X | X | X | | X | | X | | X |
| PK/PD sampling^{m} | | X | X | | X | | X | | X |
| Immunogenicity (ADA)ⁿ | | X | | | X | | X | | X |
| Review safety card^{o} | | X | X | X | X | X | X | X | X |
| Breakthrough hemolysis^{p} | | ←Monitor continuously→ | | | | | | | |
| Concomitant medications | X | ←Monitor continuously→ | | | | | | | |
| Adverse events | X | ←Monitor continuously→ | | | | | | | |
| ravulizumab administration (patients weighing < 20 kg)^{q,r} | | X^{s} | X | X | X | X | X | X | |
| ravulizumab administration (patients weighing ≥ 20 kg)^{q} | | X^{s} | X | | X | | X | | |

| | | | | | | | | | |
|---|---|---|---|---|---|---|---|---|---|
| Abbreviations: ADA = antidrug antibody; ECG = electrocardiogram; eCRF = electronic case report form; EOI = end of infusion; ET = early termination; FACIT-Fatigue = Functional Assessment of Chronic Illness Therapy-Fatigue Scale; LDH = lactate dehydrogenase; N/A = not applicable; PD = pharmacodynamics; PK = pharmacokinetics; PNH = paroxysmal nocturnal hemoglobinuria; RBC = red blood cell; WBC = white blood cell ^{a} The primary endpoint assessment is before dosing on Day 183. Dosing on Day 183 is the start of the Extension Period. Please refer to additional Day 183 post-dose assessments in Table 2. ^{b} All patients are vaccinated against meningococcal infections within 3 years prior to, or at the time of, initiating study drug. Patients who initiate study drug treatment less than 2 weeks after receiving a meningococcal vaccine receive treatment with appropriate prophylactic antibiotics until 2 weeks after vaccination. Patients who have not been vaccinated prior to initiating ravulizumab treatment receive prophylactic antibiotics prior to and for at least 2 weeks after meningococcal vaccination. Patients who cannot be vaccinated receive antibiotic prophylaxis for the entire treatment period and for 8 months following last dose. ^{c} WBC (granulocyte and monocyte) and RBC clone size measured by high-sensitivity flow cytometry at Screening and Day 1; RBC clone size only on Day 71 and Day 183. ^{d} Height at baseline and Day 183 only. Weight is collected pre-dose on dosing days. ^{e} Pregnancy testing is required only for female patients of childbearing potential (*i*.*e*., have achieved menarche). Serum pregnancy test is performed at Screening and Day 183, urine pregnancy test at all other required time points. A negative pregnancy test result is required prior to administering study drug to female patients of childbearing potential at the indicated study visits. ^{f} Investigator or designee assessment of the following events: fatigue, hemoglobinuria abdominal pain, dyspnea, dysphagia, chest pain, and erectile dysfunction. On dosing days, assessments are performed prior to dosing. ^{g} On dosing days, assessments are performed prior to dosing. Pediatric FACIT-Fatigue only in patients ≥ 5 years of age (self-reported by patients who were ≥ 8 years of age at the time of enrollment and reported by caregivers for patients who were ≥ 5 to < 8 years of age at the time of enrollment). ^{h} Abbreviated physical examination consists of a body system relevant examination based upon Investigator (or qualified designee) judgment and patient symptoms. At least one body system is checked for an abbreviated exam. ⁱ Vital sign measurements are taken after the patient has been resting for at least 5 minutes and include systolic and diastolic blood pressure (BP) (millimeters of mercury [mmHg]), heart rate (beats/minute), respiratory rate (breaths/minute), and temperature (degrees Celsius [C] or degrees Fahrenheit [°F]). On dosing days, vital signs are taken pre-dose. ^{j} Single 12-lead ECG is collected at Screening and pre-dose on Day 71 and Day 183. Patients are supine for approximately 5 to 10 minutes before ECG collection and remain supine (but awake) during ECG collection. ^{k} Clinical laboratory measurements are collected pre-dose on dosing days and not from a heparinized line. ^{l} For patients entering the study on eculizumab therapy, screening LDH is obtained within 24 hours prior to a scheduled eculizumab dose. ^{m}Serum samples for PK/PD analyses are collected at the indicated visits. For indicated visits falling on dosing days, samples are collected pre-dose (within 0.5 hours prior to the start of infusion) and at end of infusion (EOI) (within 0.5 hours after the EOI from the patient's opposite, noninfused arm). To minimize needle sticks to the patient, the pre-dose sample is drawn through the venous access created for the dose infusion, prior to administration of the dose. As noted, the post-dose sample is drawn from the opposite, non-infused arm. For indicated visits not falling on dosing days, samples are collected at any time that visit day. If a supplemental dose is administered PK/PD samples are collected at pre-dose and at end of infusion. If a loading dose is administered as two separate infusions < 24 hours apart, PK/PD samples are collected before the first infusion (*i*.*e*., the pre-dose sample) and after the second infusion (*i*.*e*., the end of infusion sample). All collection times are recorded in the eCRF. ⁿ ADA serum samples are collected pre-dose on Days 1, 71 and 127 or at any time during an ET visit. Day 183 collection occurs prior to first dose in the Extension Period. ^{o} Review the Clinical Trial Participant Safety Information Card with the patient/caregiver and discuss the importance of carrying the safety card at all times, and the risks ravulizumab treatment, including the risk of meningococcal infection. ^{p} If a suspected event of breakthrough hemolysis occurs, LDH, PK and PD parameters are to be analyzed at the central laboratory. If the suspected event of breakthrough does not occur at a scheduled visit, an unscheduled visit occurs for evaluation of the patient and collection of the required LDH, PK and PD parameters. ^{q} Dose regimen is based on body weight obtained at the study visit. If the study drug is prepared the night prior to the visit, the weight from the previous visit is used. ^{r} Should a patient's weight change from < 20 kg to ≥ 20 kg on a "q4w only" visit, the patient receives the q4w dose that day. At the patient's next q8w visit, the new q8w dose is given. ^{s} For patients entering the study on eculizumab therapy, Day 1 occurs 2 weeks from the patient's last dose of eculizumab | | | | | | | | | |

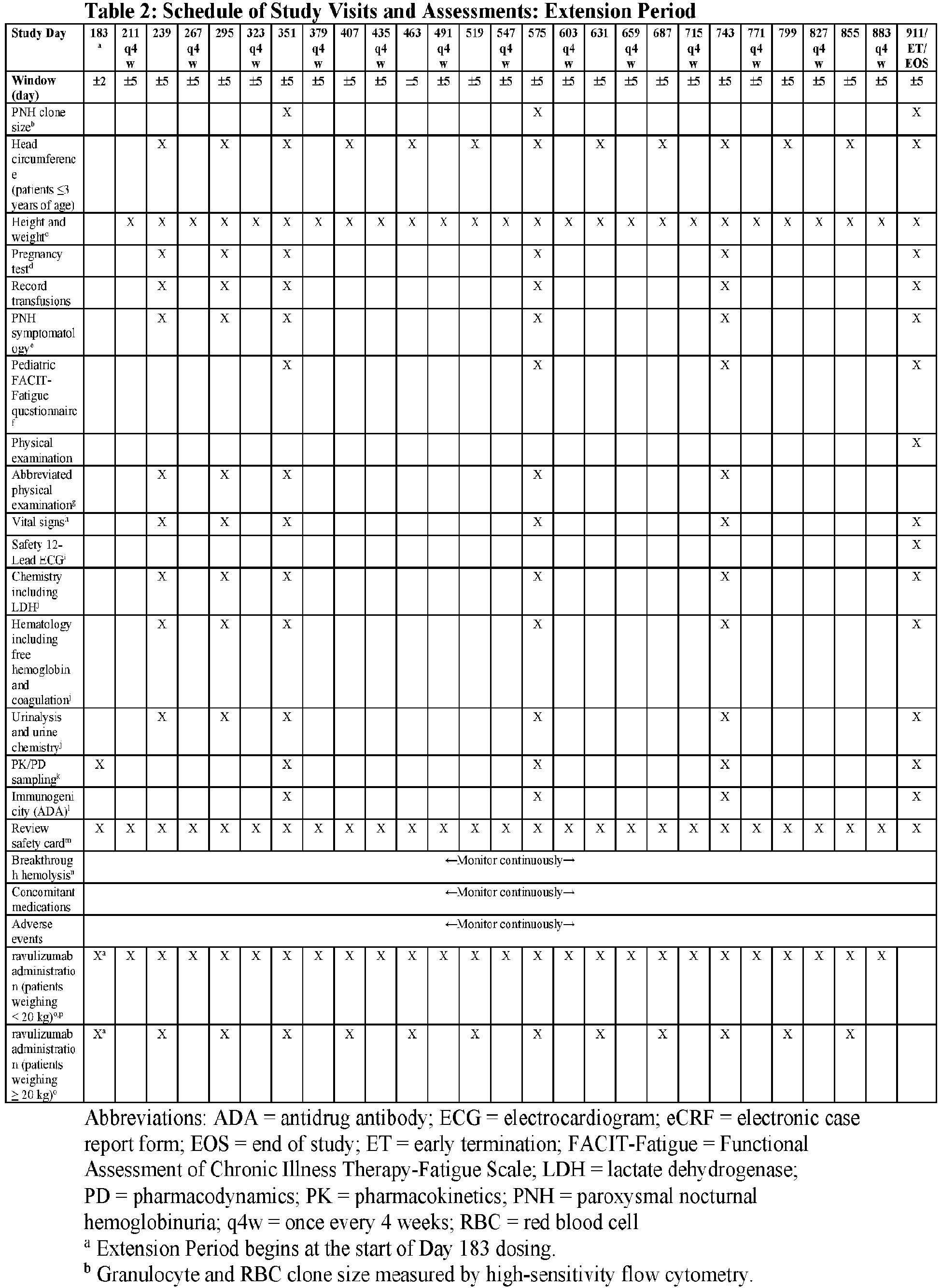

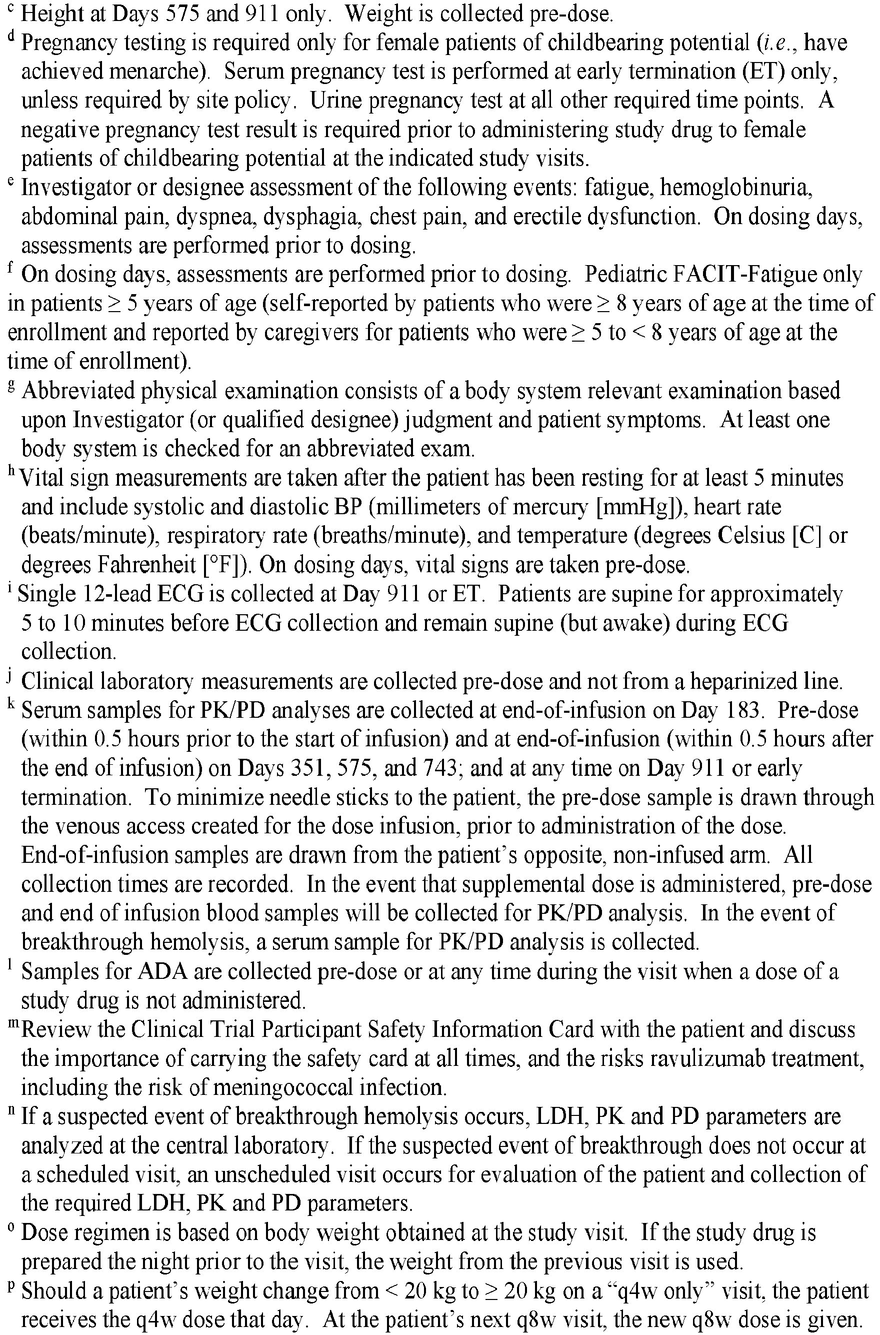

### 5. Study Population

Pediatric patients (age < 18 years) with a documented diagnosis of PNH are enrolled and treated with ravulizumab at approximately 40 investigative sites globally. Approximately 12 patients are enrolled to ensure at least 10 evaluable patients complete the 26-week period. Individuals who do not meet the criteria for participation in this study (screen failure) can be rescreened once.

Patients are eligible for enrollment in the study only if they meet all of the following criteria and none of the exclusion criteria:
1. Male and female patients < 18 years of age and weighing ≥ 5 kg at the time of consent.
2. Documented diagnosis of PNH, confirmed by high-sensitivity flow cytometry evaluation (Borowitz, M. et al., Cytometry B Clin. Cytom., 78:211-30, 2010) of red blood cells (RBCs) and white blood cells (WBCs), with granulocyte or monocyte clone size of ≥ 5%.
3. For patients not currently treated with eculizumab, presence of one or more of the following PNH-related signs or symptoms within three months of Screening: fatigue, hemoglobinuria, abdominal pain, shortness of breath (dyspnea), anemia, history of a major adverse vascular event (including thrombosis), dysphagia, or erectile dysfunction; or history of packed red blood cell (pRBC) transfusion due to PNH.
4. LDH values at Screening as follows:
   a. For patients not currently treated with eculizumab, LDH level ≥ 1.5 × ULN.
   b. For patients who are currently taking eculizumab, LDH ≤ 1.5 × ULN (sample must be obtained on a scheduled eculizumab-dosing day prior to dose administration [*i.e.,* at trough eculizumab level] and analyzed by the central laboratory).
5. To reduce the risk of meningococcal infection (*N meningitidis*), all patients must be vaccinated against meningococcal infections within three years prior to, or at the time of, initiating study drug. Patients who initiate study drug treatment less than two weeks after receiving a meningococcal vaccine receive treatment with appropriate prophylactic antibiotics until two weeks after vaccination. Patients who cannot be vaccinated receive antibiotic prophylaxis for the entire treatment period and for eight months following last dose.
6. Patients must have been vaccinated against *Haemophilus influenzae* type b (Hib) and *Streptococcus pneumoniae* according to national and local vaccination schedule guidelines, as appropriate.
7. Female patients of childbearing potential (*i*.*e*., have achieved menarche) and male patients with female partners of childbearing potential follow protocol-specified guidance for avoiding pregnancy while on treatment and for 8 months after last dose of study drug.

Patient's legal guardian gives written informed consent and the patient gives written informed assent and comply with the study visit schedule.

Patients are excluded from study enrollment if they meet any of the following criteria:
1. Platelet count < 30,000/mm³ (30 × 10⁹/L) at Screening.
2. Absolute neutrophil count < 500/µL (0.5 × 10⁹/L) at Screening.
3. History of bone marrow transplantation.
4. History of *N meningitidis* infection.
5. History of unexplained, recurrent infection.
6. Active systemic bacterial, viral, or fungal infection within 14 days prior to study drug administration on Day 1.
7. History of malignancy within 5 years of Screening with the exception of adequately treated nonmelanoma skin cancer or carcinoma in situ of the cervix.
8. History of or ongoing major cardiac, pulmonary, renal, endocrine, or hepatic disease (e.g., active hepatitis) that, in the opinion of the Investigator or Sponsor, precludes the patient's participation in an investigational clinical trial.
9. Unstable medical conditions (*e.g.,* myocardial ischemia, active gastrointestinal bleed, severe congestive heart failure, anticipated need for major surgery within 6 months of Screening, coexisting chronic anemia unrelated to PNH) that makes them unlikely to tolerate the requirements of the protocol.
10. Concomitant use of anticoagulants is prohibited if not on a stable regimen for at least 2 weeks prior to Day 1.
11. History of hypersensitivity to any ingredient contained in the study drug, including hypersensitivity to murine proteins.
12. Females who plan to become pregnant or are currently pregnant or breastfeeding.
13. Females of childbearing potential who have a positive pregnancy test result at Screening or on Day 1.
14. Participation in another interventional treatment study or use of any experimental therapy within 30 days before initiation of study drug on Day 1 in this study or within 5 half-lives of that investigational product, whichever is greater.
15. Known or suspected history of drug or alcohol abuse or dependence within 1 year prior to the start of Screening.
16. Known medical or psychological condition(s) or risk factor that, in the opinion of the Investigator or Sponsor, might interfere with the patient's full participation in the study, pose any additional risk for the patient, or confound the assessment of the patient or outcome of the study.

### 6. Study Treatment

Ravulizumab is a humanized, anti-C5 mAb produced in Chinese hamster cells. The ravulizumab drug product is supplied for clinical studies as a sterile, preservative-free 10 mg/mL solution in single-use vials and is designed for infusion by diluting into commercially available saline (0.9% sodium chloride injection; country-specific pharmacopeia) for administration via IV infusion (see Table 3).

**Table 3: Study Drug**

| | |
|---|---|
| **Product Name** | ravulizumab |
| **Dosage Form** | Concentrated solution (10 mg/mL) for infusion |
| **Route of Administration** | Intravenous infusion |
| **Physical Description** | Clear to translucent, slight whitish color, practically free from particles |
| **Manufacturer** | Alexion Pharmaceuticals, Inc. or Contracted Manufacturing Organization |

Ravulizumab is packaged in US Pharmacopeia/European Pharmacopeia Type 1 borosilicate glass vials and stoppered with a butyl rubber stopper with an aluminum overseal and a flip-off cap. Study drug is supplied in kits.

Upon arrival of the study drug kits at the study site, the pharmacist or designee promptly removes the study drug kits from the shipping cooler and stores them in their original cartons under refrigerated conditions at 2C to 8C (35°F to 47°F) and protected from light. Ravulizumab is not frozen. Study drug is stored in a secure, limited-access storage area, and the temperature is monitored daily.

The admixed drug product is at room temperature prior to administration. The material is not heated (*e.g.,* by using a microwave or other heat source) other than by ambient air temperature.

Ravulizumab is not administered as an intravenous (IV) push or bolus injection. Infusions of study drug are prepared using aseptic technique. The patient's required dose of ravulizumab is further diluted into commercially available saline (0.9% sodium chloride; country-specific pharmacopeia) at the volume specified in Table 4. Ravulizumab admixture is administered to the patient using an IV tubing administration set via an infusion pump. Use of a 0.2 micron filter is required during infusion of ravulizumab.

**Table 4: Dosing Reference Chart for Ravulizumab Dose Preparation**

| **Dose Type** | **Body Weight (kg)^{a}** | **Dose (mg)** | **ravulizumab Volume (mL)** | **Saline Volum e (mL)** | **Total Volume (mL)** | **Minimum Infusion Duration minutes (hours)** | **Maximum Infusion Rate (mL/hour)** |
|---|---|---|---|---|---|---|---|
| Loading | ≥ 5 to < 10 | 600 | 60 | 60 | 120 | 228 (3.8) | 31.5 |
| | ≥ 10 to < 20 | 600 | 60 | 60 | 120 | 113 (1.9) | 63.1 |
| | ≥ 20 to < 30 | 900 | 90 | 90 | 180 | 86 (1.5) | 120.0 |
| | ≥ 30 to < 40 | 1200 | 120 | 120 | 240 | 77 (1.3) | 184.6 |
| | ≥ 40 to < 60 | 2400 | 240 | 240 | 480 | 114 (1.9) | 253 |
| | ≥ 60 to < 100 | 2700 | 270 | 270 | 540 | 102(1.7) | 318 |
| | ≥ 100 | 3000 | 300 | 300 | 600 | 108 (1.8) | 333 |
| Maintenance | ≥ 5 to < 10 | 300 | 30 | 30 | 60 | 113 (1.9) | 31.5 |
| | ≥ 10 to < 20 | 600 | 60 | 60 | 120 | 113 (1.9) | 63.1 |
| | ≥ 20 to < 30 | 2100 | 210 | 210 | 420 | 194 (3.3) | 127.2 |
| | ≥ 30 to < 40 | 2700 | 270 | 270 | 540 | 167 (2.8) | 192.8 |
| | ≥ 40 to < 60 | 3000 | 300 | 300 | 600 | 140 (2.4) | 250 |
| | ≥ 60 to < 100 | 3300 | 330 | 330 | 660 | 120 (2.0) | 330 |
| | ≥ 100 | 3600 | 360 | 360 | 720 | 132 (2.2) | 328 |

| | | | | | | | |
|---|---|---|---|---|---|---|---|
| ^{a} Body weight is obtained at the study visit. If the study drug is prepared the night prior to the visit, the weight from the previous visit is used. | | | | | | | |

Doses of study drug are only prepared and dispensed by qualified study personnel. Study drug is dispensed only to enrolled patients who are confirmed eligible for participation. Once study drug is prepared for a patient, it is only administered to that patient. Vials of study drug are for one-time use only and any drug product remaining in the vial is not used for another patient. Any drug remaining in the infusion tubing or infusion bag is not used for another patient.

Patients receive a loading dose of ravulizumab on Day 1, followed by maintenance dosing of ravulizumab on Day 15 and once every eight weeks (q8w) thereafter for patients weighing ≥ 20 kg, or once every four weeks (q4w) for patients weighing < 20 kg, as shown in Table 5. With the agreement of the Medical Monitor, the 600 mg loading dose can be given to patients weighing ≥ 5 to < 10 kg as two separate infusions administered no more than 24 hours apart. In addition, administration of a supplemental dose of ravulizumab is permitted. Dosages are based on the patient's body weight recorded on the day of dosing or the most recently recorded weight. For patients entering the study on eculizumab therapy, Day 1 of study treatment occurs 2 weeks from the patient's last dose of eculizumab.

**Table 5: Loading and Maintenance Treatment Regimens**

| **Body Weight Range (kg)^{a}** | **Loading Dose (mg)** | **Maintenance Doses (mg)** | **Maintenance Dosing Frequency** |
|---|---|---|---|
| ≥ 5 to < 10 | 600^{b} | 300 | q4w |
| ≥ 10 to < 20 | 600 | 600 | q4w |
| ≥ 20 to < 30 | 900 | 2100 | q8w |
| ≥ 30 to < 40 | 1200 | 2700 | q8w |
| ≥ 40 to < 60 | 2400 | 3000 | q8w |
| ≥ 60 to < 100 | 2700 | 3300 | q8w |
| > 100 | 3000 | 3600 | q8w |

| | | | |
|---|---|---|---|
| Abbreviations: q4w = once every 4 weeks; q8w = once every 8 weeks ^{a} Dose regimen is based on body weight obtained at the study visit. If the study drug is prepared the night prior to the visit, the weight from the previous visit is used. ^{b} The 600 mg loading dose can be given to patients weighing > 5 to < 10 kg as two separate infusions administered no more than 24 hours apart. | | | |

After the Primary Evaluation Period, all patients roll over into an Extension Period and continue their weight-based maintenance dose of ravulizumab on Day 183 and once every eight weeks (q8w) thereafter for patients weighing ≥ 20 kg, or once every four weeks (q4w) for patients weighing < 20 kg, until the product is registered or approved (in accordance with country specific regulation) or for up to 2 years, whichever occurs first.

The actual time of all dose administrations, including any supplemental dose or dose administered as two separate infusions, is recorded. Due to its mechanism of action, the use of ravulizumab increases the patient's susceptibility to meningococcal infection (N. *meningitidis*). To reduce the risk of meningococcal infection, all patients are vaccinated against meningococcal infections within three years prior to, or at the time of, initiating study drug. Patients who initiate study drug treatment less than two weeks after receiving a meningococcal vaccine receive treatment with appropriate prophylactic antibiotics until two weeks after vaccination. Vaccines against serotypes A, C, Y, W135 and B, where available, are recommended to prevent common pathogenic meningococcal serotypes. Patients are vaccinated or revaccinated according to current national vaccination guidelines or local practice for vaccination use with eculizumab. Vaccination may not be sufficient to prevent meningococcal infection. Consideration is given per official guidance and local practice on the appropriate use of antibacterial agents and vaccination. All patients are monitored for early signs of meningococcal infection, evaluated immediately if infection is suspected, and treated with appropriate antibiotics, if necessary. Patients are also vaccinated against Hib and *S pneumoniae* according to national and local vaccination schedule guidelines.

### 7. Efficacy Assessments

Administration of a packed red blood cell (pRBC) transfusion, including the hemoglobin result and symptoms that triggered the transfusion, and the number of units transfused, are documented in the electronic case report form (eCRF).

Blood and urine samples are collected at the times indicated in the Schedule of Assessments.

The following disease-related laboratory parameters are measured during the study: LDH, free hemoglobin, occult blood, urine, total C5, haptoglobin, reticulocyte count, PNH RBC clone size evaluated by high-sensitivity flow cytometry, and estimated glomerular filtration rate (calculated using the Schwartz formula).

The Investigator or designee record for each patient the presence or absence of the following signs and symptoms of PNH: fatigue, chest pain, hemoglobinuria, abdominal pain, dyspnea, dysphagia, erectile dysfunction, and red/dark urine or hemoglobinuria.

The Pediatric FACIT-Fatigue scale is a 13-item questionnaire that assesses fatigue and its impact upon daily activities and function over the preceding 7 days. Each item is scored on a 5-point scale, and total scores range from 0 to 52, with higher score indicating better QoL. The questionnaire is self-reported by patients who were ≥ 8 years of age at the time of enrollment and reported by caregivers for patients who were ≥ 5 to < 8 years of age at the time of enrollment. Patients < 5 years of age are assessed. The Pediatric FACIT-Fatigue Scale is shown in Table 6.

**Table 6: Pediatric FACIT-Fatigue Scale**

| | None of the time | A little bit of the time | Some of the time | Most of the time | All of the time |
|---|---|---|---|---|---|
| I feel tired.............................. | 0 | 1 | 2 | 3 | 4 |
| I have energy (or strength)........................... | 0 | 1 | 2 | 3 | 4 |
| I could do my usual things at home............... | 0 | 1 | 2 | 3 | 4 |
| I had trouble starting things because I was too tired................................................... | 0 | 1 | 2 | 3 | 4 |
| I had trouble finishing things because I was too tired................................................... | 0 | 1 | 2 | 3 | 4 |
| I needed to sleep during the day.................. | 0 | 1 | 2 | 3 | 4 |
| I got upset by being too tired to do the things I want to do............................................ | 0 | 1 | 2 | 3 | 4 |
| Being tired made it hard for me to play or go out with my friends as much as I'd like.......... | 0 | 1 | 2 | 3 | 4 |
| I needed help doing my usual things at home... | 0 | 1 | 2 | 3 | 4 |
| I feel weak........................................... | 0 | 1 | 2 | 3 | 4 |
| I was too tired to eat................................. | 0 | 1 | 2 | 3 | 4 |
| Being tired made me sad........................... | 0 | 1 | 2 | 3 | 4 |
| Being tired made me mad (angry)............... | 0 | 1 | 2 | 3 | 4 |

Major adverse vascular events (MAVEs) are assessed as part of the planned evaluation for adverse events (AEs). The description of the MAVE, including the method of diagnosis (*e*.*g*., magnetic resonance imaging, ultrasound, angiogram), date of diagnosis, and date resolved (or ongoing) is collected on the eCRF as part of the patient's medical history (prior to baseline). A MAVE is defined as follows: thrombophlebitis/deep vein thrombosis, pulmonary embolus, myocardial infarction, transient ischemic attack, unstable angina, renal vein thrombosis, acute peripheral vascular occlusion, mesenteric/visceral vein thrombosis or infarction, mesenteric/visceral arterial thrombosis or infarction, hepatic/portal vein thrombosis (Budd-Chiari syndrome), cerebral arterial occlusion/cerebrovascular accident, cerebral venous occlusion, renal arterial thrombosis, gangrene (non-traumatic; non-diabetic), amputation (non-traumatic; non-diabetic), and dermal thrombosis.

### 8. Safety Assessments

A review of demographic parameters, including age, gender, race and ethnicity is performed. A complete medical history is taken and documented. Weight and height are recorded. Head circumference is recorded for patients who are ≤ 3 years of age. The patient's PNH medical history, including PNH symptoms, date of diagnosis, PNH clone size, pRBC transfusions and history of any MAVEs, are documented at the Screening visit.

The patient's medical history, including prior and concomitant conditions/disorders and transfusion history, is recorded at the Screening Visit. Medication (prescription or over the counter, including vitamins and/or herbal supplements) use within 28 days prior to the start of Screening is also be recorded. Details of prior treatment with eculizumab, including dose level and frequency, are collected. Meningococcal vaccination within 3 years prior to the first dose of study drug, and vaccination history for Hib and *S pneumoniae* from birth, is also recorded.

A physical examination includes the following assessments: general appearance; skin; head, ear, eye, nose, and throat; neck; lymph nodes; chest; heart; abdominal cavity; limb; central nervous system; and musculoskeletal system. An abbreviated physical examination consists of a body system relevant examination based upon Investigator judgment and patient symptoms. Physical growth (height, weight and head circumference [the latter only in patients ≤ 3 years of age]) is assessed.

Vital sign measurements are taken after the patient has been resting for at least five minutes, and include systolic and diastolic blood pressure (BP; millimeters of mercury [mmHg]), heart rate (beats/minute), respiratory rate (breaths/minute), and temperature (degrees Celsius [C] or degrees Fahrenheit [°F]).

Samples for serum pregnancy, hematology, chemistry, coagulation, and urinalysis are performed at the times specified in the Schedule of Assessments. Specific laboratory assessments are provided in Table 7. Samples for laboratory assessments are collected before each study drug administration. An alternative blood sampling schedule for infants, for whom less blood volume is collected, must be used. If a suspected event of breakthrough hemolysis occurs, an unscheduled visit takes place at which a sample is collected for analysis of LDH and PK/PD by the central laboratory.

**Table 7: Laboratory Assessments**

| **Hematology** | | **Clinical Chemistry** | |
|---|---|---|---|
| | Free hemoglobin | | Alanine aminotransferase |
| | Haptoglobin | | Albumin |
| | Hematocrit | | Alkaline phosphatase |
| | Hemoglobin | | Aspartate aminotransferase |
| | Mean corpuscular hemoglobin | | Bicarbonate |
| | Platelet count | | Blood urea nitrogen |
| | RBC count | | Calcium |
| | RBC distribution width | | Chloride |
| | RBC mean corpuscular volume | | Creatinine |
| | Reticulocyte count | | Gamma-glutamyltransferase |
| | WBC count | | Glucose |
| | WBC differential | | Lactate dehydrogenase |
| | | | Magnesium |
| **Coagulation Panel** | | | Phosphorus |
| | International normalized ratio | | Potassium |
| | Partial thromboplastin time | | Sodium |
| | Prothrombin time | | Total bilirubin (direct and indirect) |
| | | | Total protein |
| **Urinalysis** | | | Uric acid |
| | Appearance | | |
| | Bilirubin | **Other** | |
| | Blood | | Antidrug antibody |
| | Color | | Beta human chorionic gonadotropin (*females of childbearing potential only*) |
| | Glucose | | |
| | Ketone | | Chicken RBC assay |
| | Nitrite | | Free and total C5 |
| | pH | | Pharmacokinetic assay |
| | Specific gravity | | PNH clone size |
| | Urobilinogen | | |
| | Urine Chemistry | | |
| | Microalbumin | | |

| | | | |
|---|---|---|---|
| Abbreviations: C5 = complement component 5; PNH = paroxysmal nocturnal hemoglobinuria; RBC = red blood cell; WBC = white blood cell | | | |

It is anticipated that some laboratory values may be outside the normal value range due to the underlying disease. The Investigators should use their medical judgment when assessing the clinical significance of these values. Clinical significance is defined as any variation in laboratory measurements that has medical relevance and that results in a change in medical care. If clinically significant laboratory changes from baseline value are noted, the changes are documented as adverse events. The Investigator also assesses the relationship to study treatment for all clinically significant out-of-range values. The Investigator monitors the patient through additional laboratory assessments until (1) values have returned to the normal range or baseline level, or (2) in the judgment of the Investigator, values that are outside the normal range are not related to the administration of study drug or other protocol-specific procedures.

For females of childbearing potential (*i*.*e*., have achieved menarche), a serum or urine pregnancy test (beta-human chorionic gonadotropin [β-hCG]) is performed according to the Schedule of Assessments.

Blood samples are analyzed for the hematology parameters listed in Table 7. Blood samples are also analyzed for the serum chemistry parameters listed in Table 7. Indirect bilirubin is calculated from total and direct bilirubin values. Therefore, indirect bilirubin results are not available if direct bilirubin is below the limit of quantification.

Chemistry assessments are performed at the time points specified in the Schedule of Assessments. The estimated glomerular filtration rate is calculated using the Schwartz formula for all visits at which serum chemistries are collected. Blood samples are also analyzed for the coagulation parameters listed in Table 7.

Urine samples are analyzed for the parameters listed in Table 7. A microscopic examination of urine samples is performed if the results of the macroscopic analysis are abnormal.

For each patient, single 12-lead digital electrocardiograms (ECGs) are collected according to the Schedule of Assessments. Patients are supine for approximately 5 to 10 minutes before ECG collection and remain supine (but awake) during ECG collection. The ECG is assessed to see whether it is within normal limits and to determine the clinical significance of the results.

Blood samples are collected to test for presence and titer of anti drug antibodies to ravulizumab in serum prior to study drug administration as indicated in the Schedule of Assessments. Further characterization of antibody responses is conducted as appropriate, including binding and neutralizing antibodies, PK/PD, safety and activity of ravulizumab.

An adverse event is any untoward medical occurrence in a patient administered a pharmaceutical product that does not necessarily have a causal relationship with this treatment. An adverse event can therefore, be any unfavorable or unintended sign (e.g., an abnormal laboratory finding), symptom, or disease temporally associated with the use of a medicinal product, whether or not considered related to the medicinal product.

Situations in which an untoward medical occurrence did not occur (*e.g.,* hospitalization for elective surgery if planned before the start of the study, admissions for social reasons or for convenience), and anticipated day-to-day fluctuations of pre-existing disease(s) or condition(s) present or detected at the start of the study that do not worsen are not adverse events. Transfusions are treated as efficacy endpoints. Transfusions administered in the inpatient or outpatient setting should not be captured as adverse events or serious adverse events unless identified as such. Lack of drug effect is not an adverse event in clinical studies, because the purpose of the clinical study is to establish drug effect.

A medication error (including intentional misuse, abuse, and overdose of the product) or use other than what is defined in the protocol is not considered an adverse event unless there is an untoward medical occurrence as a result of a medication error.

Cases of pregnancy that occur during maternal or paternal exposure to investigational product are to be reported within 24 hours of Investigator/site awareness. Data on fetal outcome and breastfeeding is collected for regulatory reporting and safety evaluation.

The severity of adverse events is graded using Common Terminology Criteria for Adverse Events (CTCAE) version 4.03 or higher. A grading (severity) scale is provided for each adverse event term. Each CTCAE term is a Lowest Level Term (LLT) per the Medical Dictionary for Regulatory Activities (MedDRA^{®}). Each LLT is coded to a MedDRA preferred term (PT). Grade refers to the severity of the adverse event. The CTCAE assigns a grade of 1 through 5, with unique clinical descriptions of severity for each adverse event, as set forth in Table 8.

**Table 8: Adverse Event Severity Grading Scale**

| **Grade** | **Description** |
|---|---|
| Grade 1 | Mild; asymptomatic or mild symptoms; clinical or diagnostic observations only; intervention not indicated |
| Grade 2 | Moderate; minimal, local or noninvasive intervention indicated; limiting age-appropriate instrumental ADL^{a} |
| Grade 3 | Severe or medically significant, but not immediately life-threatening; hospitalization or prolongation of hospitalization indicated; disabling; limiting self-care ADL^{b} |
| Grade 4 | Life-threatening consequences; urgent intervention indicated. |
| Grade 5 | Death related to AE. |

| | |
|---|---|
| Abbreviations: ADL = activities of daily living; AE = adverse event ^{a} Instrumental ADL refers to preparing meals, shopping for groceries or clothes, using the telephone, managing money, etc. ^{b} Self-care ADL refers to bathing, dressing and undressing, feeding self, using the toilet, taking medications, and not bedridden. | |

Severity and seriousness are differentiated. Severity describes the intensity of an adverse event, while the term seriousness refers to an adverse event that has met specific criteria for a serious adverse event. A causality assessment (Unrelated, Unlikely, Possible, Probable, or Definite) is provided for all adverse events (both serious and nonserious) based upon the Investigator's medical judgment and the observed symptoms associated with the event, as set forth in Table 9.

**Table 9: Causality Assessment Descriptions**

| **Assessment** | **Description** |
|---|---|
| Not Related/Unrelated | Suggests that there is no causal association between the investigational product and the reported event. |
| Unlikely Related | Suggests that the clinical picture is highly consistent with a cause other than the investigational product but attribution cannot be made with absolute certainty and a relationship between the investigational product and AE cannot be excluded with complete confidence. |
| Possibly Related | Suggests that treatment with the investigational product may have caused or contributed to the AE (*i.e.,* the event follows a reasonable temporal sequence from the time of drug administration and/or follows a known response pattern to the investigational product, but could also have been produced by other factors). |
| Probably Related | Suggests that a reasonable temporal sequence of the event with the investigational product administration exists and the likely causal association of the event with the investigational product. This is be based upon the known pharmacological action of the investigational product, known or previously reported adverse reactions to the investigational product or class of drugs, or judgment based on the Investigator's clinical experience. |
| Definitely Related | Temporal relationship to the investigational product, other conditions (concurrent illness, concurrent medication reaction, or progression/expression of disease state) do not appear to explain event, corresponds with the known pharmaceutical profile, improvement on discontinuation, reappearance on rechallenge. |

A SAE is any untoward medical occurrence that: results in death, is life-threatening (*i*.*e*., patient was at risk of death at the time of the event), requires inpatient hospitalization or prolongation of existing hospitalization, results in persistent or significant disability/incapacity, or is a congenital anomaly/birth defect.

Important medical events that may not result in death, be immediately life-threatening, or require hospitalization are considered a serious adverse event when, based upon appropriate medical judgment, they jeopardize the patient or require intervention to prevent one of the outcomes listed above.

Suspected unexpected serious adverse reactions (SUSARs) are serious events that are not listed in the Investigator's Brochure and that the Investigator identifies as related to investigational product or procedure.

All adverse events are collected from the signing of the informed consent form (ICF) until 56 days after the last dose of study drug for patients with ET or until 56 days after the last dose of study drug for patients who complete the study. All adverse events are recorded on the eCRF upon becoming aware of their occurrence. Investigators are instructed to report the serious adverse event, including their assessment (e.g., severity, seriousness, and potential relatedness to study drug).

### 9. Pharmacokinetics (PK) and Pharmacodynamics (PD)

Blood samples for determination of serum drug concentrations and PD assessments are collected before and after administration of study drug at the time points indicated in the Schedule of Assessments. The actual date and time (24-hour clock time) of each sampling is recorded. In the event of breakthrough hemolysis, an additional PK/PD sample is required.

End of infusion blood samples for PK and PD assessment is collected from the arm opposite to the arm used for infusing drug. Assessments for PK/PD are as follows: serum ravulizumab concentration over time, free and total C5 concentrations over time, and cRBC hemolytic activity over time

### 10. Statistical Methods and Planned Analyses

A clinical study report (CSR) is produced based on efficacy, safety, PK, PD and immunogenicity data collected through the end of the 26-week Primary Evaluation Period (Day 183). A final CSR to summarize long-term efficacy, safety, PK, PD and immunogenicity parameters is produced at study completion.

This study is descriptive in nature and not statistically powered for hypothesis testing due to the rarity of disease in pediatric patients. Approximately 12 patients are enrolled to ensure at least 10 evaluable patients complete the 26-week period. A sample size of 10 is expected to be sufficient to adequately describe PK/PD in pediatric patients with PNH. Efficacy analyses are performed on the Full Analysis Set (FAS). The FAS includes all patients who received at least one dose of ravulizumab and have at least one post-baseline assessment.

Safety analyses are performed on the Safety Set, defined as all patients who receive at least one dose of ravulizumab.

Pharmacokinetic and PD analyses are performed on all patients who receive at least one dose of ravulizumab and who have evaluable PK and PD data.

Patient demographic and baseline characteristics, including medical history and transfusion history, are summarized for the clinical study report (FAS) and Safety sets. All patients are included in the summaries of patient disposition, which describe the frequency and percentage of patients screened and treated and who completed or withdraw from the study, along with reason for withdrawal from the study.

The numbers of patients who are treated, discontinue treatment (along with reason for treatment discontinuation), complete or withdraw from the Primary Evaluation Period (along with reason for withdrawal), enter the Extension Period, and complete or withdraw from the Extension Period (along with reason for withdrawal) are tabulated.

Each patient's prior and concomitant medication use is coded using the World Health Organization Drug Dictionary, and the frequency and percentage of concomitant medications is summarized. Medications are summarized by Anatomic-Therapeutic-Chemical class and generic name using frequency counts and percentages of patients in the Safety set. The number of infusions received per patient is tabulated for the FAS and Safety sets. Efficacy analyses is performed using the FAS. Continuous variables are summarized using descriptive statistics, including number of observations and mean, SD, median, minimum and maximum values. Categorical variables are summarized by frequency counts and percentage of patients. Analyses are conducted separately for naive and previously eculizumab-treated patients.

The following definitions are used for adverse events. A pretreatment adverse event is any adverse event that starts after providing informed consent, but before the first infusion of study drug. A treatment-emergent adverse event is any adverse event that starts during or after the first infusion of study drug. A treatment-emergent serious adverse event is a treatment-emergent AE (TEAE) that is serious.

The incidence of TEAEs, TEAEs leading to withdrawal from the study, TEAEs leading to study treatment discontinuation, drug-related TEAEs, TEAEs during study drug administration, severe TEAEs and SAEs are summarized. All adverse events are coded using MedDRA version 18 or higher, and summarized by system organ class and preferred term. Detailed by-patient listings of TEAEs, SAEs, related TEAEs, TEAEs during study drug administration, TEAEs leading to withdrawal from the study and TEAEs leading to study treatment discontinuation are provided.

Adverse changes from baseline in physical examination findings are classified as adverse events and analyzed accordingly.

Vital signs are summarized descriptively at baseline and postbaseline time points and for changes from baseline by treatment group.

Height, weight and head circumference (the latter only for patients ≤ 3 years of age) are summarized descriptively at baseline and post-baseline time points and for changes from baseline by treatment group.

Changes in clinical chemistry, hematology, and urinalysis results from baseline to post-baseline study time points are summarized descriptively. Shift tables over time are presented for all central laboratory values, where applicable, using normal, low or high based on normal range values. Listings of patients with abnormal results are provided. By-patient data listings of ECG parameters are provided. Changes from baseline in electrocardiogram intervals (PR, RR, QT and QTcF) are provided. QT interval is corrected for heart rate using Fridericia's formula (QTcF).

Incidence and titers for antidrug antibodies (ADAs) to ravulizumab are summarized in tabular format. Individual serum concentration data for all patients who receive at least one dose of ravulizumab and who have evaluable PK data are used to derive PK parameters for ravulizumab.

Graphs of mean serum concentration-time profiles are constructed. Graphs of serum concentration-time profiles for individual patients are also provided. Actual dose administration and sampling times is used for all calculations. Descriptive statistics are calculated for serum concentration data at each sampling time, as appropriate. Assessment of population-PK is considered using data from this study or in combination with data from other studies.

PD analyses are performed for all patients who receive at least one dose of ravulizumab and who have evaluable PD data.

Descriptive statistics are presented for all ravulizumab PD endpoints at each sampling time. The PD effects of ravulizumab administered IV are evaluated by assessing the absolute values and changes and percentage changes from baseline in total and free C5 serum concentrations and change from baseline in cRBC hemolysis over time, as appropriate.

Assessments of ravulizumab PK/PD relationships are explored using data from this study or in combination with data from other studies. Analyses are conducted separately for naive and previously eculizumab-treated patients.

If a Day 1 assessment is missing, the Screening assessment is used as the baseline assessment.

### EXAMPLE 2: Interim Results from Phase 3, Open-Label Study of Ravulizumab in Children and Adolescents with PNH

An open label study of ravulizumab in a PNH pediatric patient population was conducted substantially according to the protocol described above in Example 1 to assess pharmacokinetics (PK), pharmacodynamics (PD), safety, and efficacy.

### A. Study Design

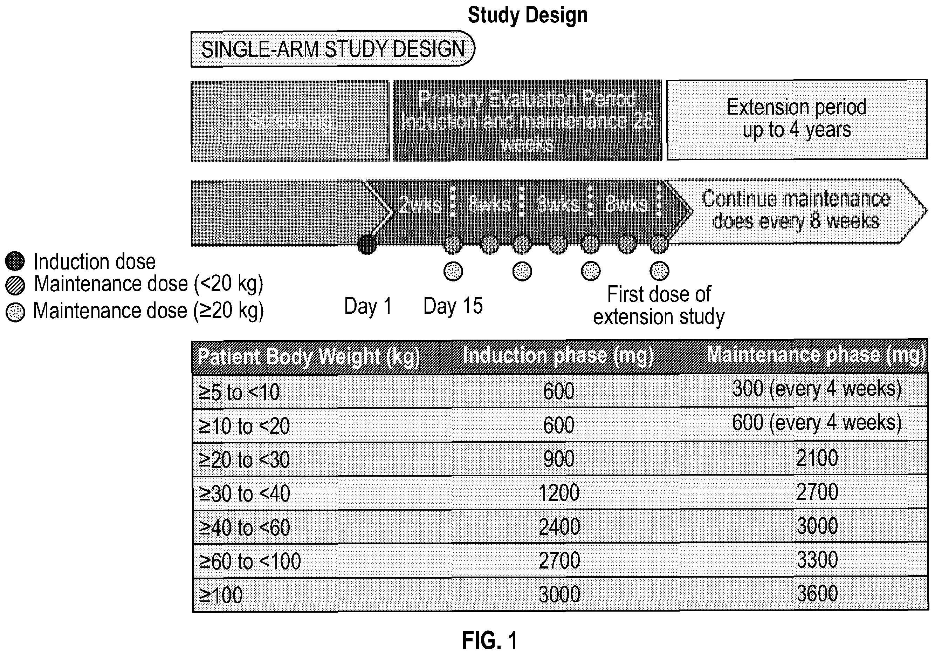

The study design is set forth in FIG. 1. Thirteen patients were enrolled and 12 were included in this interim analysis. All patients were vaccinated against Haemophilus influenzae type b (Hib) and Streptococcus pneumoniae. At screening, LDH < 1.5xULN at for patients taking eculizumab and ≥ 1.5xULN for patients not taking eculizumab. Patients had a granulocyte or monocyte PNH clone size > 5%.

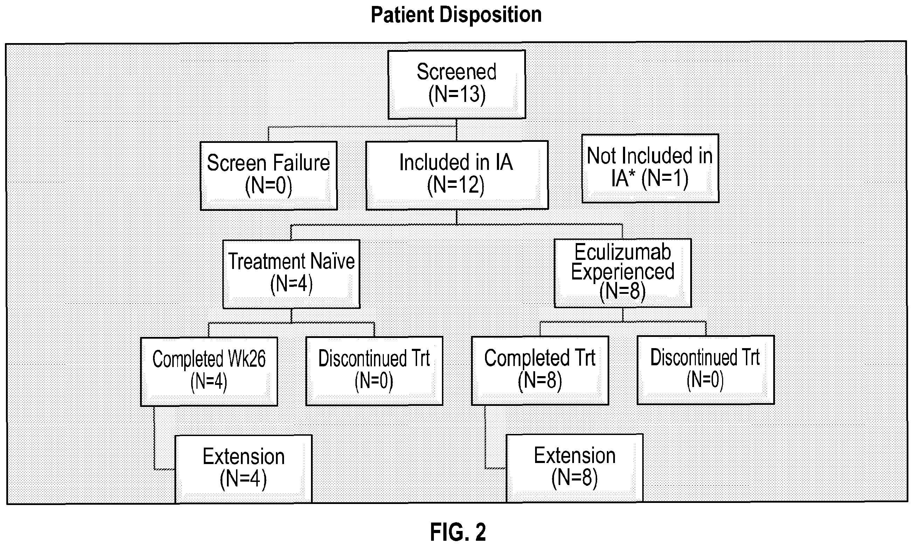

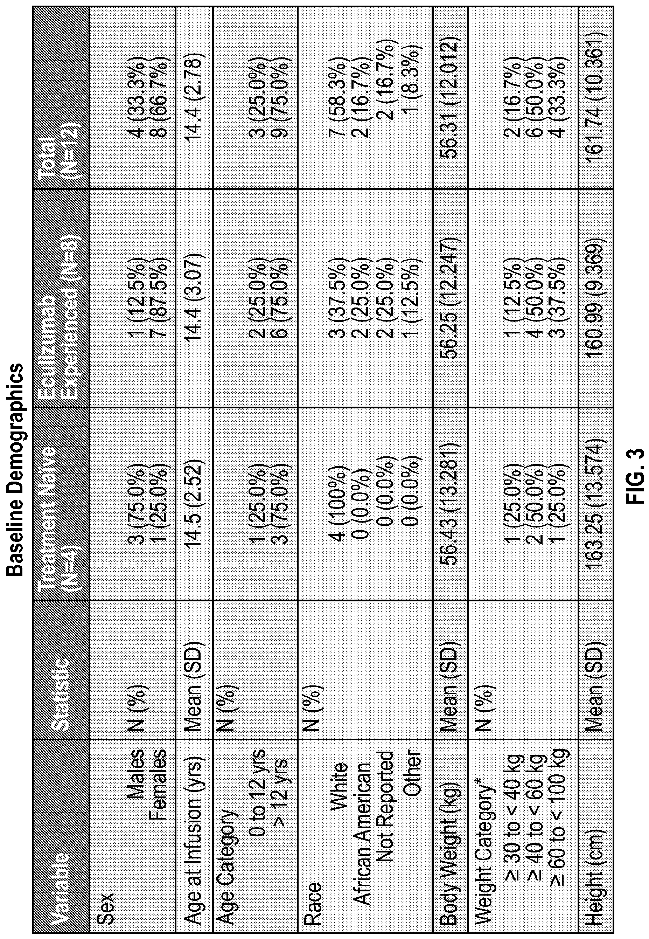

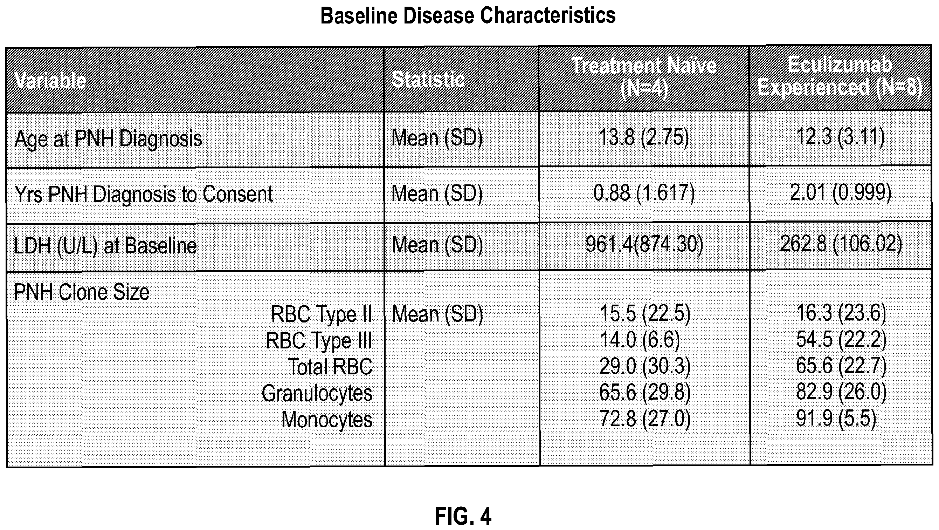

No patient discontinued in the primary evaluation period. An overview of patient disposition is set forth in FIG. 2. Baseline patient demographics are set forth in FIG. 3 and baseline disease characteristics are set forth in FIG. 4.

### B. Results

Following weight-based ravulizumab dosing in pediatric patients with PNH, Cmax, Ctrough, free C5, and cRBC hemolytic activity were consistent with previous pharmacology studies of ravulizumab at Weeks 2, 10, 18, and 26.

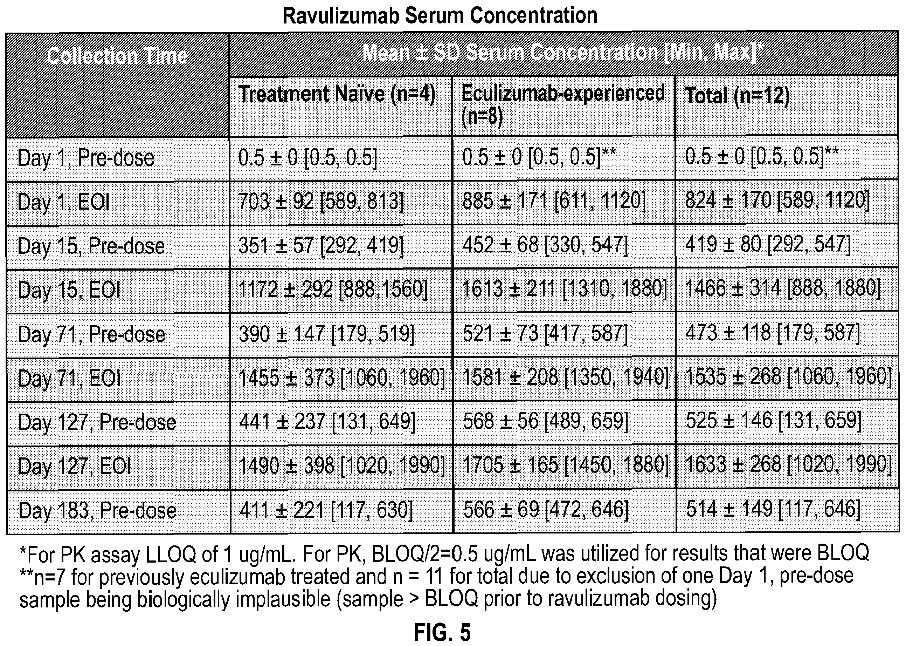

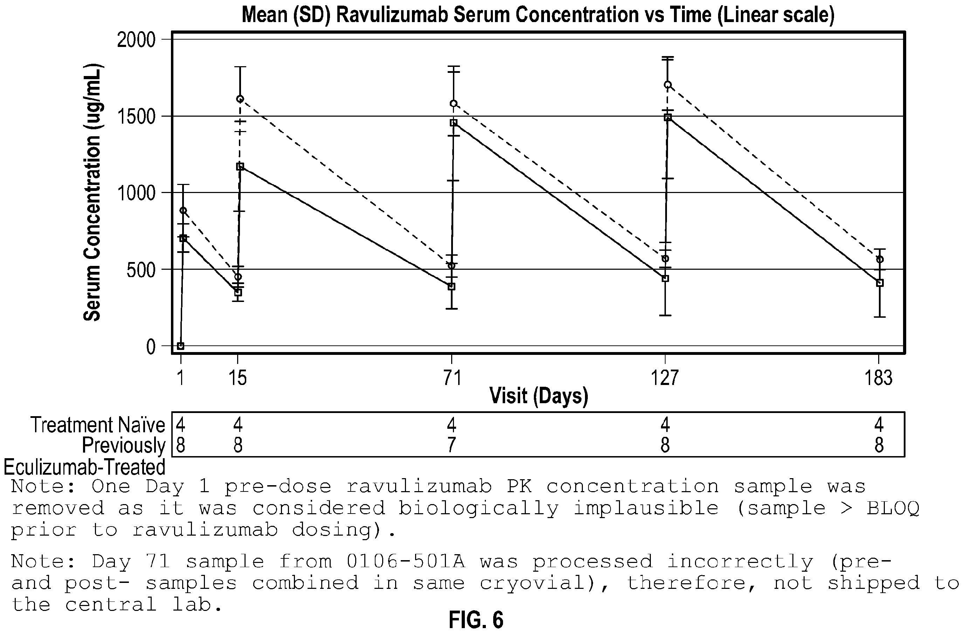

Ravulizumab serum concentrations and mean serum concentrations over time for treatment naive and eculizumab experienced patients are set forth in FIGs. 5 and 6, respectively. All Ctrough values were > 175 µg/mL, excluding one patient during the study. The one excluded treatment naive patient experienced infection (SAE), MAVE(SAE) and received multiple packed red blood cell (pRBC) transfusions during the study, which resulted in two Ctrough values below 175 µg/mL. Despite sub-therapeutic concentrations, free C5 control was maintained in this patient throughout the primary evaluation period.

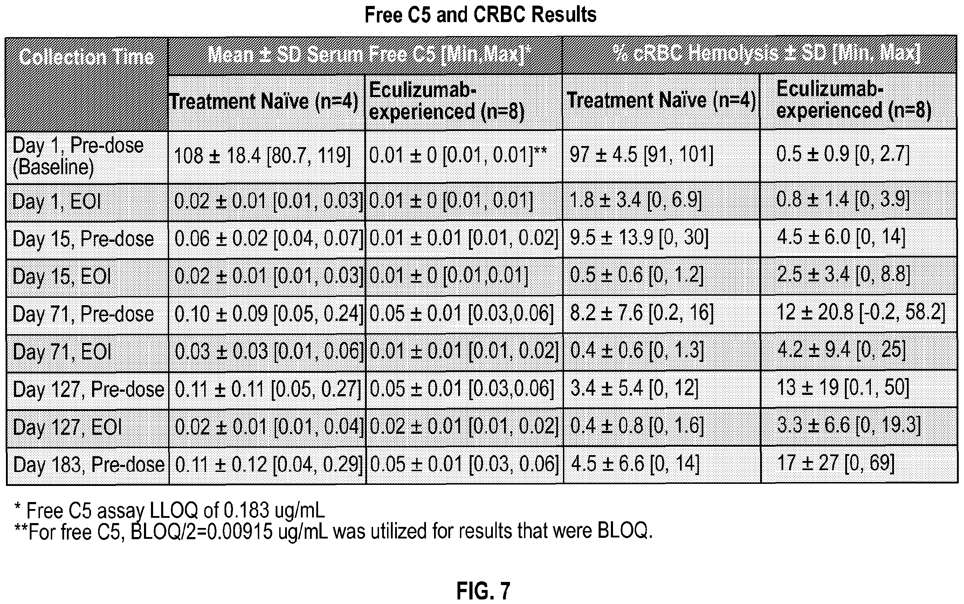

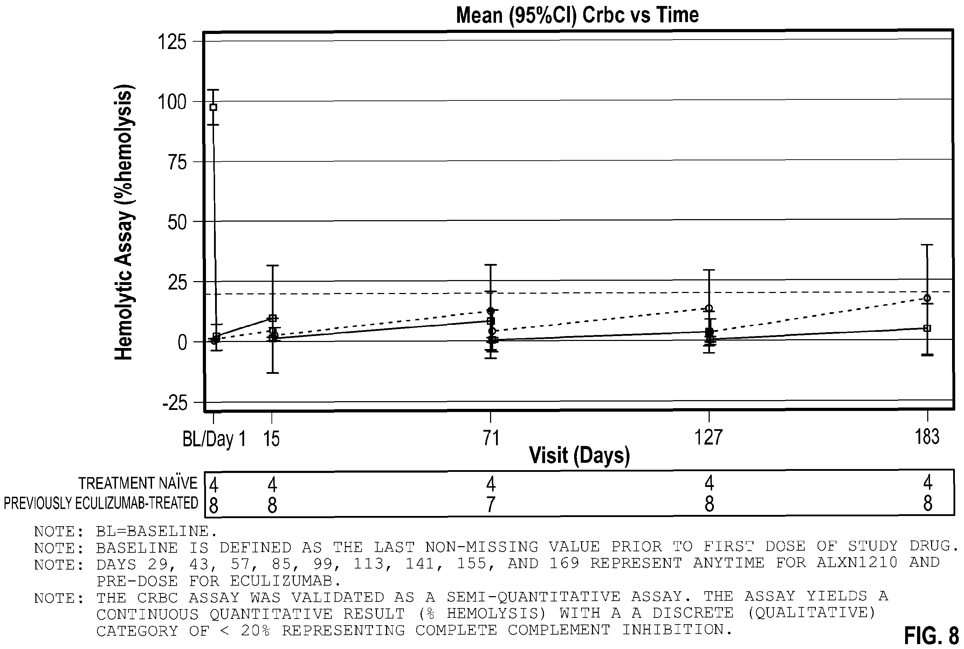

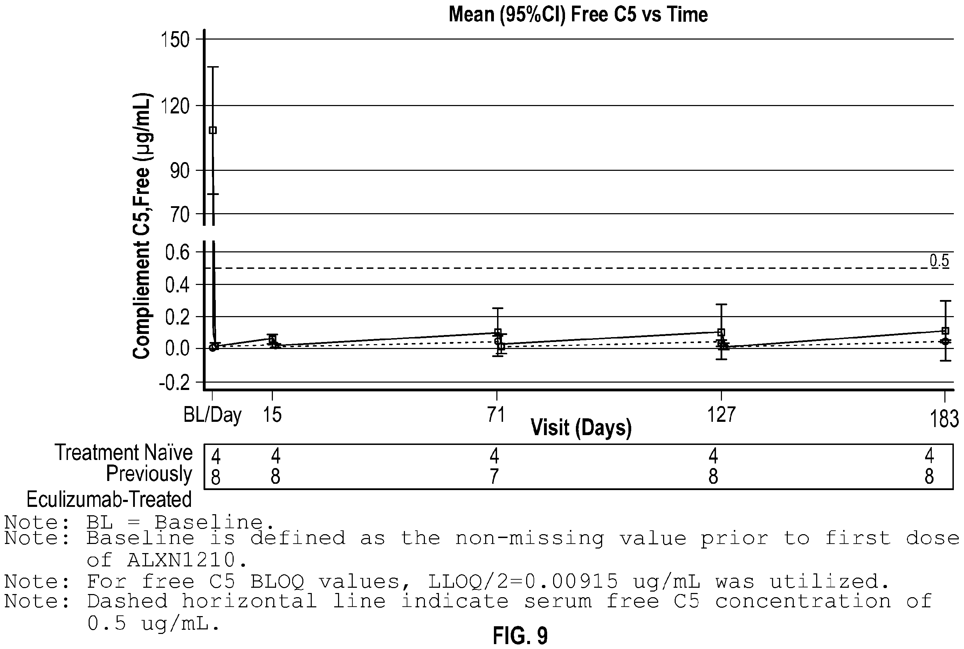

Free C5 and CRBC results over time for treatment naive and eculizumab experienced patients are shown in FIG. 7. Mean cRBC and mean free C5 over time for both patients groups are set forth in FIGs. 8 and 9, respectively. All serum free C5 values were < 0.5 µg/mL (i.e., the threshold for complete terminal complement inhibition). Mean cRBC hemolytic activity was below 20% (i.e., the threshold for complete inhibition of hemolysis) at all timepoints for both cohorts. No treatment emergent antidrug antibody (ADA) was observed.

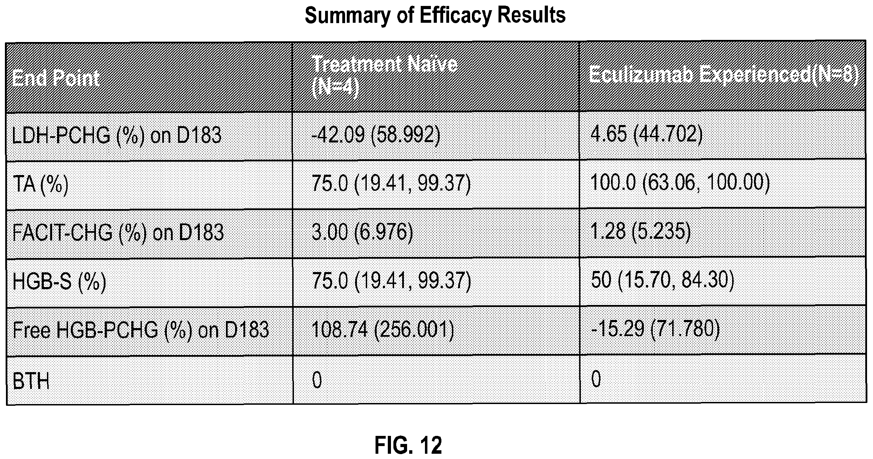

With respect to efficacy, descriptive statistics of endpoints demonstrate efficacy of ravulizumab in treatment naive and eculizumab treated PNH pediatric patients. No patients experienced breakthrough hemolysis (BTH). The overall efficacy results are set forth in FIG. 12. For purposes of this study, the following definitions were used. LDH Percent Change from Baseline (LDH-PCHG) is defined as percentage change in LDH from Baseline to Day 183. Transfusion Avoidance (TA) is defined as the proportion of patients who remain transfusion-free and do not require a transfusion per protocol-specified guidelines through Day 183. Facet Fatigue (FACIT) is defined as change in quality of life (QoL) assessed via the Pediatric Functional Assessment of Chronic Illness Therapy (FACIT)-Fatigue Scale, from Baseline to Day 183. The proportion of patients with stabilized hemoglobin (HGB-S) is defined as avoidance of a ≥ 2 g/dL decrease in hemoglobin level from baseline in the absence of transfusion through Day 183. Free Hemoglobin Percent Change from Baseline (Free HGB-PCHG) is defined as the percentage change in Free Hemoglobin from Baseline to Day 183. The proportion of patients with breakthrough hemolysis is defined as at least one new or worsening symptom or sign of intravascular hemolysis (fatigue, hemoglobinuria, abdominal pain, shortness of breath [dyspnea], anemia [hemoglobin < 10 g/dL], major adverse vascular event [MAVE, including thrombosis], dysphagia, or erectile dysfunction) in the presence of elevated LDH ≥ 2 × upper limit of normal [ULN], after prior LDH reduction to < 1.5 × ULN on therapy for patients who enter the study naive to complement inhibitor treatment; For patients who enter the study stabilized on eculizumab treatment, elevated LDH ≥ 2 × ULN.

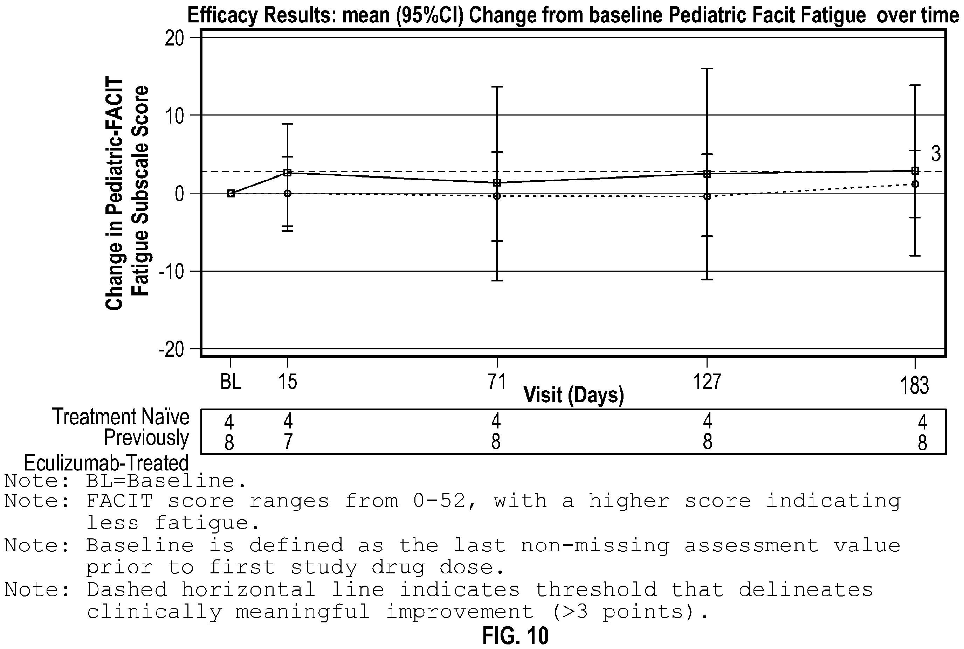

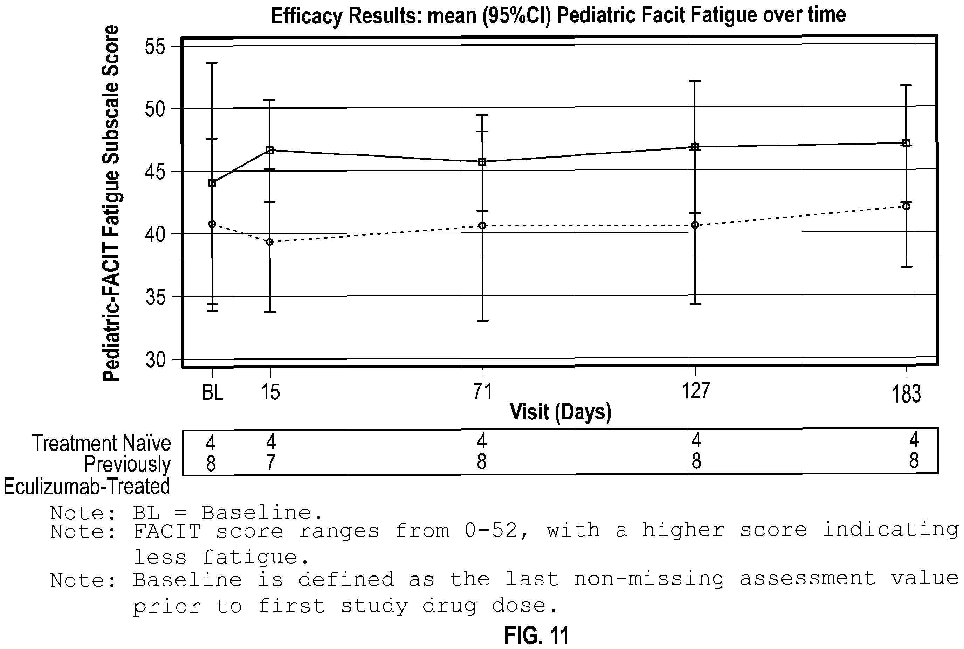

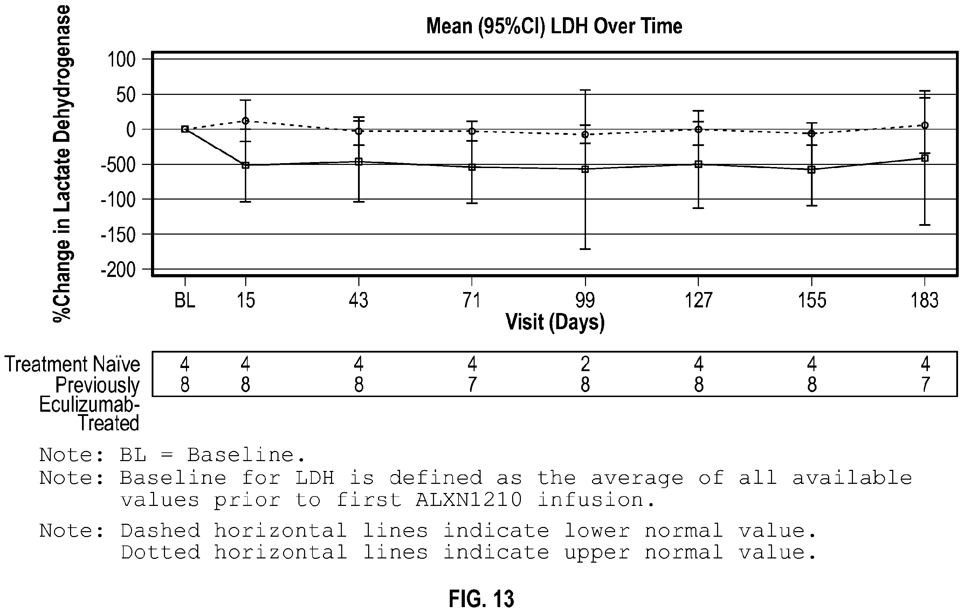

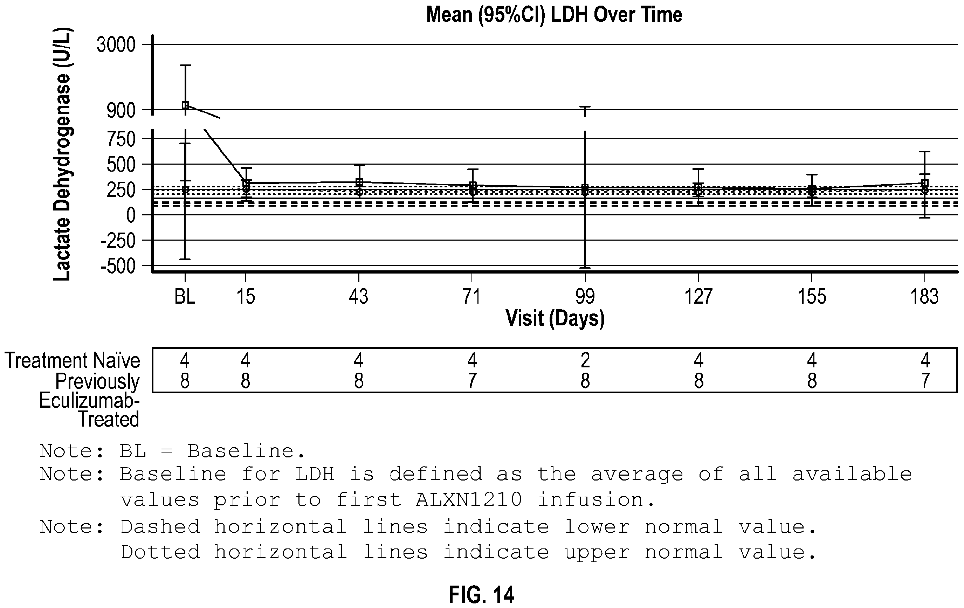

The mean change from baseline pediatric FACIT Fatigue Score and mean FACIT Fatigue Score over time for treatment naive and eculizumab experienced patients is set forth in FIGs. 10 and 11, respectively. The mean (95% CI) % change and mean LDH over time for both patient groups are set forth in FIGs. 13 and 14, respectively. The LDH % change from Baseline (mean (SD)) for Treatment Naive patients was -42.09 (58.992) and 4.65 (44.702) for Eculizumab Experienced patients.

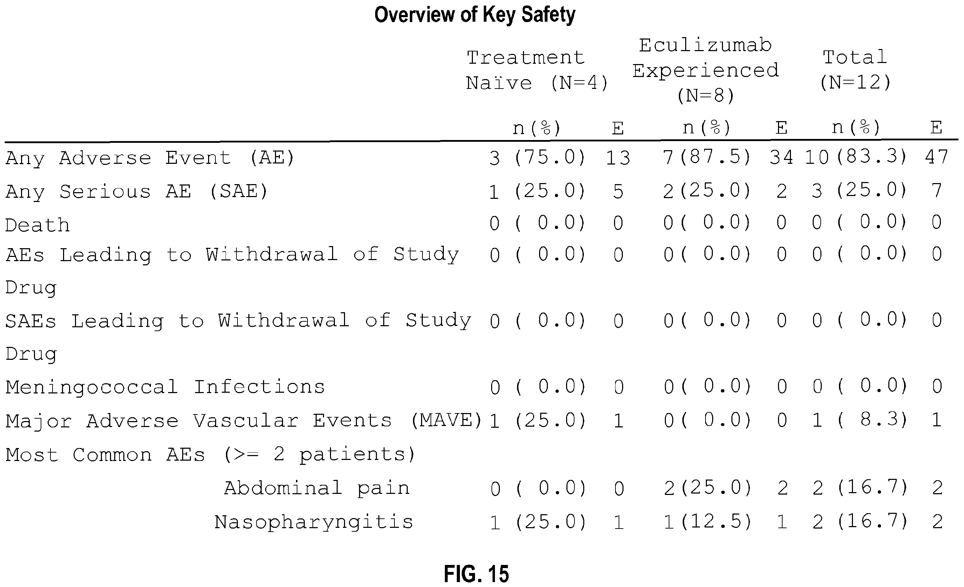

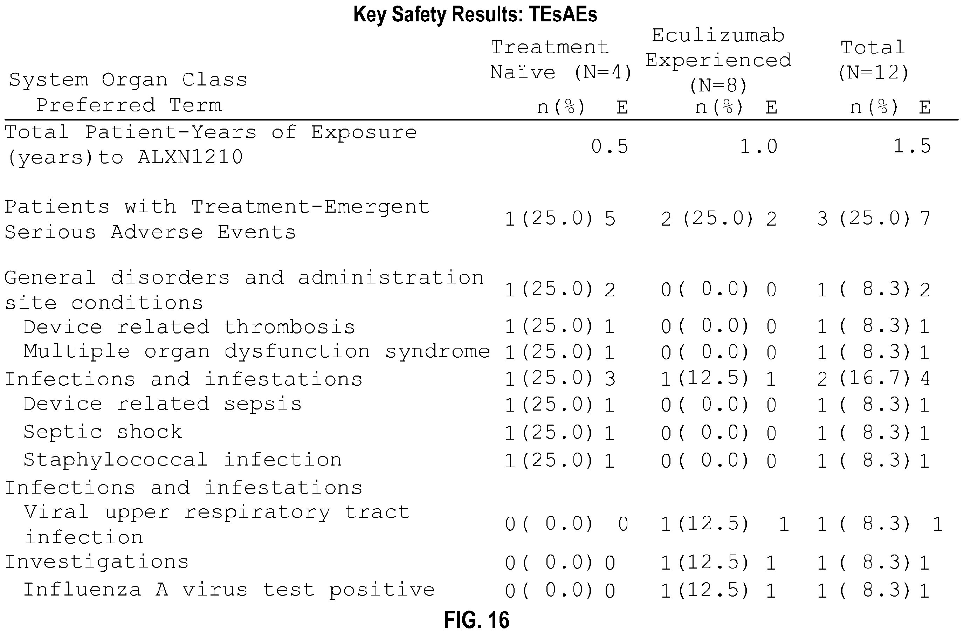

Ravulizumab administration in pediatric PNH patients was safe and well tolerated. An overview of key safety points is set forth in FIG. 15. Treatment-emergent serious adverse events (TESAES) are summarized in FIG. 16. The most frequent adverse events (AEs) were abdominal pain and nasopharyngitis (2 patients each). No meningococcal infection, discontinuations due to AEs, or deaths were observed during primary evaluation period. One patient had a Major Adverse Vascular Event (MAVE).

In sum, pharmacokinetic (PK)/pharmacodynamic (PD), efficacy, and safety of ravulizumab in treatment naive and eculizumab experienced pediatric patients was consistent with previous adult study results (i.e., ALXN1210-PNH-301 (Clinical trials ID: NCT02946463) and ALXN1210-PNH-302 (Clinical trials ID: NCT03056040) studies).

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used. The materials, methods, and examples are illustrative only and not intended to be limiting. All publications, patent applications, patents, sequences, database entries (e.g., PUBMED, NCBI, UNIPROT, Clinical Trials accession numbers), and other references mentioned herein are incorporated by reference in their entirety. In case of conflict, the present specification, including definitions, will control.

While particular embodiments of the present disclosure have been illustrated and described, it would be obvious to those skilled in the art that various other changes and modifications may be made without departing from the spirit and scope of the disclosure. It is therefore intended to cover in the appended claims all such changes and modifications that are within the scope of this disclosure.

### SEQUENCE SUMMARY

| |
|---|
| SEQ ID NO:1 |
| GYIFSNYWIQ |
| SEQ ID NO:2 |
| EILPGSGSTEYTENFKD |
| SEQ ID NO:3 |
| YFFGSSPNWYFDV |
| SEQ ID NO:4 |
| GASENIYGALN |
| SEQ ID NO:5 |
| GATNLAD |
| SEQ ID NO:6 |
| QNVLNTPLT |
| SEQ ID NO:7 |
| |
| SEQ ID NO:8 |
| |
| SEQ ID NO:9 |
| ASTKGPSVFP LAPCSRSTSE STAALGCLVK DYFPEPVTVS WNSGALTSGV HTFPAVLQSS GLYSLSSVVT VPSSNFGTQT YTCNVDHKPS NTKVDKTVER KCCVECPPCP APPVAGPSVF LFPPKPKDTL MISRTPEVTC VVVDVSQEDP EVQFNWYVDG VEVHNAKTKP REEQFNSTYR VVSVLTVLHQ DWLNGKEYKC KVSNKGLPSS IEKTISKAKG QPREPQVYTL PPSQEEMTKN QVSLTCLVKG FYPSDIAVEW ESNGQPENNY KTTPPVLDSD GSFFLYSRLT VDKSRWQEGN VFSCSVMHEA LHNHYTQKSL SLSLGK |
| SEQ ID NO:10 |
| QVQLVQSGAE VKKPGASVKV SCKASGYIFS NYWIQWVRQA PGQGLEWMGE ILPGSGSTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVECPP CPAPPVAGPS VFLFPPKPKD TLMISRTPEV TCVVVDVSQE DPEVQFNWYV DGVEVHNAKT KPREEQFNST YRVVSVLTVL HQDWLNGKEY KCKVSNKGLP SSIEKTISKA KGQPREPQVY TLPPSQEEMT NQVSLTCLVK GFYPSDIAVE WESNGQPENN YKTTPPVLDS DGSFFLYSRL TVDKSRWQEG NVFSCSVMHE ALHNHYTQKS LSLSLGK |
| SEQ ID NO:11 |
| |
| SEQ ID NO:12 |
| QVQLVQSGAE VKKPGASVKV SCKASGHIFS NYWIQWVRQA PGQGLEWMGE ILPGSGHTEY TENFKDRVTM TRDTSTSTVY MELSSLRSED TAVYYCAR FGSSPNWYFD VWGQGTLVTV SS |
| **SEQ ID NO:13** |
| ASTKGPSVFP LAPCSRSTSE STAALGCLVK DYFPEPVTVS WNSGALTSGV HTFPAVLQSS GLYSLSSVVT VPSSNFGTQT YTCNVDHKPS NTKVDKTVER KCCVECPPCP APPVAGPSVF LFPPKPKDTL MISRTPEVTC VVVDVSQEDP EVQFNWYVDG VEVHNAKTKP REEQFNSTYR VVSVLTVLHQ DWLNGKEYKC KVSNKGLPSS IEKTISKAKG QPREPQVYTL PPSQEEMTKN QVSLTCLVKG FYPSDIAVEW ESNGQPENNY KTTPPVLDSD GSFFLYSRLT VDKSRWQEGN VFSCSVLHEA LHSHYTQKSL SLSLGK |
| **SEQ ID NO:14** |
| QVQLVQSGAE VKKPGASVKV SCKASGHIFS NYWIQWVRQA PGQGLEWMGE ILPGSGHTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVECPP CPAPPVAGPS VFLFPPKPKD TLMISRTPEV TCVVVDVSQE DPEVQFNWYV DGVEVHNAKT KPREEQFNST YRVVSVLTVL HQDWLNGKEY KCKVSNKGLP SSIEKTISKA KGQPREPQVY TLPPSQEEMT KNQVSLTCLV KGFYPSDIAV EWESNGQPEN NYKTTPPVLD SDGSFFLYSR LTVDKSRWQE GNVFSCSVLH EALHSHYTQK SLSLSLGK |
| **SEQ ID NO:15** |
| ASTKGPSVFP LAPCSRSTSE STAALGCLVK DYFPEPVTVS WNSGALTSGV HTFPAVLQSS GLYSLSSVVT VTSSNFGTQT YTCNVDHKPS NTKVDKTVER KCCVECPPCP APPVAGPSVF LFPPKPKDTL YITREPEVTC VVVDVSHEDP EVQFNWYVDG MEVHNAKTKP REEQFNSTFR VVSVLTVVHQ DWLNGKEYKC KVSNKGLPAP IEKTISKTKG QPREPQVYTL PPSREEMTKN QVSLTCLVKG FYPSDIAVEW ESNGQPENNY KTTPPMLDSD GSFFLYSKLT VDKSRWQQGN VFSCSVMHEA LHNHYTQKSL SLSPGK |
| **SEQ ID NO:16** |
| QVQLVQSGAE VKKPGASVKV SCKASGYIFS NYWIQWVRQA PGQGLEWMGE ILPGSGSTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVTSSNFGT QTYTCNVDHK PSNTKVDKTV ERKCCVECPP CPAPPVAGPS VFLFPPKPKD TLYITREPEV TCVVVDVSHE DPEVQFNWYV DGMEVHNAKT KPREEQFNST FRVVSVLTVV HQDWLNGKEY KCKVSNKGLP APIEKTISKT KGQPREPQVY TLPPSREEMT KNQVSLTCLV KGFYPSDIAV EWESNGQPEN NYKTTPPMLD SDGSFFLYSK LTVDKSRWQQ GNVFSCSVMH EALHNHYTQK SLSLSPGK |
| **SEQ ID NO:17** |
| GASENIYHALN |
| **SEQ ID NO:18** |
| EILPGSGHTEYTENFKD |
| **SEQ ID NO:19** |
| GHIFSNYWIQ |
| **SEQ ID NO:20** |
| QVQLVQSGAE VKKPGASVKV SCKASGHIFS NYWIQWVRQA PGQGLEWMGE ILPGSGHTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVECPP CPAPPVAGPS VFLFPPKPKD TLMISRTPEV TCVVVDVSQE DPEVQFNWYV DGVEVHNAKT KPREEQFNST YRVVSVLTVL HQDWLNGKEY KCKVSNKGLP SSIEKTISKA KGQPREPQVY |
| TLPPSQEEMT KNQVSLTCLV KGFYPSDIAV EWESNGQPEN NYKTTPPVLD SDGSFFLYSR LTVDKSRWQE GNVFSCSVMH EALHNHYTQK SLSLSLGK |
| **SEQ ID NO:21** |
| SYAIS |
| **SEQ ID NO:22** |
| GIGPFFGTANYAQKFQG |
| **SEQ ID NO:23** |
| DTPYFDY |
| **SEQ ID NO:24** |
| SGDSIPNYYVY |
| **SEQ ID NO:25** |
| DDSNRPS |
| **SEQ ID NO:26** |
| QSFDSSLNAEV |
| **SEQ ID NO:27** |
| QVQLVQSGAE VKKPGSSVKV SCKASGGTFS SYAISWVRQA PGQGLEWMGG IGPFFGTANY AQKFQGRVTI TADESTSTAY MELSSLRSED TAVYYCARDT PYFDYWGQGT LVTVSS |
| **SEQ ID NO:28** |
| DIELTQPPSV SVAPGQTARI SCSGDSIPNY YVYWYQQKPG QAPVLVIYDD SNRPSGIPER FSGSNSGNTA TLTISGTQAE DEADYYCQSF DSSLNAEVFG GGTKLTVL |
| **SEQ ID NO:29** |
| NYIS |
| **SEQ ID NO:30** |
| IIDPDDSYTEYSPSFQG |
| **SEQ ID NO:31** |
| YEYGGFDI |
| **SEQ ID NO:32** |
| SGDNIGNSYVH |
| **SEQ ID NO:33** |
| KDNDRPS |
| **SEQ ID NO:34** |
| GTYDIESYV |
| **SEQ ID NO:35** |
| EVQLVQSGAE VKKPGESLKI SCKGSGYSFT NYISWVRQMP GKGLEWMGII DPDDSYTEYS PSFQGQVTIS ADKSISTAYL QWSSLKASDT AMYYCARYEY GGFDIWGQGT LVTVSS |
| **SEQ ID NO:36** |
| SYELTQPPSV SVAPGQTARI SCSGDNIGNS YVHWYQQKPG QAPVLVIYKD NDRPSGIPER FSGSNSGNTA TLTISGTQAE DEADYYCGTY DIESYVFGGG TKLTVL |
| **SEQ ID NO:37** |
| SSYYVA |
| **SEQ ID NO:38** |
| AIYTGSGATYKASWAKG |
| **SEQ ID NO:39** |
| DGGYDYPTHAMHY |
| **SEQ ID NO:40** |
| QASQNIGSSLA |
| **SEQ ID NO:41** |
| GASKTHS |
| **SEQ ID NO:42** |
| QSTKVGSSYGNH |
| **SEQ ID NO:43** |
| QVQLVESGGG LVQPGGSLRL SCAASGFTSH SSYYVAWVRQ APGKGLEWVG AIYTGSGATY KASWAKGRFT ISKDTSKNQV VLTMTNMDPV DTATYYCASD GGYDYPTHAM HYWGQGTLVT VSS |
| **SEQ ID NO:44** |
| DVVMTQSPSS LSASVGDRVT ITCQASQNIG SSLAWYQQKP GQAPRLLIYG ASKTHSGVPS RFSGSGSGTD FTLTISSLQP EDVATYYCQS TKVGSSYGNH FGGGTKVEIK |
| **SEQ ID NO:45** |
| QVQLVESGGG LVQPGRSLRL SCAASGFTVH SSYYMAWVRQ APGKGLEWVG AIFTGSGAEY KAEWAKGRVT ISKDTSKNQV VLTMTNMDPV DTATYYCASD AGYDYPTHAM HYWGQGTLVT VSSASTKGPS VFPLAPSSKS TSGGTAALGC LVKDYFPEPV TVSWNSGALT SGVHTFPAVL QSSGLYSLSS VVTVPSSSLG TQTYICNVNH KPSNTKVDKK VEPKSCDKTH TCPPCPAPEL RRGPKVFLFP PKPKDTLMIS RTPEVTCVVV DVSHEDPEVK FNWYVDGVEV HNAKTKPREE QYNSTYRVVS VLTVLHQDWL NGKEYKCKVS NKGLPSSIEK TISKAKGQPR EPQVYTLPPS REEMTKNQVS LTCLVKGFYP SDIAVEWESN GQPENNYKTT PPVLDSDGSF FLYSKLTVDK SRWQQGNVFS CSVLHEALHA HYTRKELSLS P |
| **SEQ ID NO:46** |
| DIQMTQSPSS LSASVGDRVT ITCRASQGIS SSLAWYQQKP GKAPKLLIYG ASETESGVPS RFSGSGSGTD FTLTISSLQP EDFATYYCQN TKVGSSYGNT FGGGTKVEIK RTVAAPSVFI FPPSDEQLKS GTASVVCLLN NFYPREAKVQ WKVDNALQSG NSQESVTEQD SKDSTYSLSS TLTLSKADYE KHKVYACEVT HQGLSSPVTK SFNRGEC |
| **SEQ ID NO:47** |
| QVQLQESGPGLVKPSETLSLTCTVSGDSVSSSYWTWIRQPPGKGLEWIGYIYYSGSSN YNPSLKSRATISVDTSKNQFSLKLSSVTAADTAVYYCAREGNVDTTMIFDYWGQGTLV TVSS |
| **SEQ ID NO:48** |
| AIQMTQSPSSLSASVGDRVTITCRASQGIRNDLGWYQQKPGKAPKLLIYAASSLQSGVP SRFAGRGSGTDFTLTISSLQPEDFATYYCLQDFNYPWTFGQGTKVEIK |
| **SEQ ID NO:49** |
| QVQLQESGPGLVKPSETLSLTCTVSGDSVSSSYWTWIRQPPGKGLEWIGYIYYSGSSNY NPSLKSRATISVDTSKNQFSLKLSSVTAADTAVYYCAREGNVDTTMIFDYWGQGTLVTV SSASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPPCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPS QEEMTKNQVSLTCLVKGFYPSDIAVEWESNGQPENNYKTTPPVLDSDGSFFLYSRLTVD KSRWQEGNVFSCSVMHEALHNHYTQKSLSLSLGK |
| **SEQ ID NO:50** |
| AIQMTQSPSSLSASVGDRVTITCRASQGIRNDLGWYQQKPGKAPKLLIYAASSLQSGVP SRFAGRGSGTDFTLTISSLQPEDFATYYCLQDFNYPWTFGQGTKVEIKRTVAAPSVFIF PPSDEQLKSGTASVVCLLNNFYPREAKVQWKVDNALQSGNSQESVTEQDSKDSTYSLSS |
| TLTLSKADYEKHKVYACEVTHQGLSSPVTKSFNRGEC |

### ASPECTS OF THE INVENTION

1. A method of treating a human pediatric patient with Paroxysmal Nocturnal Hemoglobinuria (PNH), the method comprising administering to the patient an effective amount of an anti-C5 antibody or antigen binding fragment thereof, comprising CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively, wherein the anti-C5 antibody or antigen binding fragment thereof, is administered:
   (a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
   (b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.
2. A method of treating a human pediatric patient with Paroxysmal Nocturnal Hemoglobinuria (PNH), the method comprising administering to the patient an effective amount of an anti-C5 antibody or antigen binding fragment thereof comprising CDR1, CDR2 and CDR3 heavy chain sequences as set forth in SEQ ID NOs:19, 18 and 3, respectively, CDR1, CDR2 and CDR3 light chain sequences as set forth in SEQ ID NOs:4, 5 and 6, respectively, and a variant human Fc constant region that binds to human neonatal Fc receptor (FcRn), wherein the variant human Fc constant region comprises Met429Leu and Asn435Ser substitutions at residues corresponding to methionine 428 and asparagine 434 of a native human IgG Fc constant region, each in EU numbering, wherein the anti-C5 antibody or antigen binding fragment thereof is administered:
   (a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
   (b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.
3. The method of aspect 1 or 2, wherein the anti-C5 antibody comprises a heavy chain variable region set forth in SEQ ID NO:12 and a light chain variable region set forth in SEQ ID NO:8.
4. The method of any one of the preceding aspects, wherein the anti-C5 antibody further comprises a heavy chain constant region set forth in SEQ ID NO:13.
5. The method of any one of the preceding aspects, wherein the antibody comprises a heavy chain polypeptide comprising the amino acid sequence set forth in SEQ ID NO:14 and a light chain polypeptide comprising the amino acid sequence set forth in SEQ ID NO:11.
6. The method of any one of the preceding aspects, wherein the anti-C5 antibody binds to human C5 at pH 7.4 and 25C with an affinity dissociation constant (K_{D}) that is in the range 0.1 nM ≤ K_{D} ≤ 1 nM.
7. The method of any one of the preceding aspects, wherein the anti-C5 antibody binds to human C5 at pH 6.0 and 25C with a K_{D} ≥ 10 nM.
8. The method of any one of the preceding aspects, wherein the anti-C5 antibody is administered to a patient weighing ≥ 5 to < 10 kg:
   (a) once on Day 1 at a dose of 600 mg; and
   (b) on Day 15 and every four weeks thereafter at a dose of 300 mg.
9. The method of any one of the preceding aspects, wherein the anti-C5 antibody is administered to a patient weighing ≥ 10 to < 20 kg:
   (a) once on Day 1 at a dose of 600 mg; and
   (b) on Day 15 and every four weeks thereafter at a dose of 600 mg.
10. The method of any one of the preceding aspects, wherein the anti-C5 antibody is administered to a patient weighing ≥ 20 to < 30 kg:
   (a) once on Day 1 at a dose of 900 mg; and
   (b) on Day 15 and every eight weeks thereafter at a dose of 2100 mg.
11. The method of any one of the preceding aspects, wherein the anti-C5 antibody is administered to a patient weighing ≥ 30 to < 40 kg:
   (a) once on Day 1 at a dose of 1200 mg; and
   (b) on Day 15 and every eight weeks thereafter at a dose of 2700 mg.
12. The method of any one of the preceding aspects, wherein the anti-C5 antibody is administered to a patient weighing ≥ 40 to < 60 kg:
   (a) once on Day 1 at a dose of 2400 mg; and
   (b) on Day 15 and every eight weeks thereafter at a dose of 3000 mg.
13. The method of any one of the preceding aspects, wherein the anti-C5 antibody is administered to a patient weighing ≥ 60 to < 100 kg:
   (a) once on Day 1 at a dose of 2700 mg; and
   (b) on Day 15 and every eight weeks thereafter at a dose of 3300 mg.
14. The method of any one of the preceding aspects, wherein the anti-C5 antibody is administered to a patient weighing ≥ 100 kg:
   (a) once on Day 1 at a dose of 3000 mg; and
   (b) on Day 15 and every eight weeks thereafter at a dose of 3600 mg.
15. The method of any one of the preceding aspects, wherein the treatment maintains a serum trough concentration of the anti-C5 antibody of 100 µg/mL or greater.
16. The method of any one of the preceding aspects, wherein the treatment maintains a serum trough concentration of the anti-C5 antibody of 200 µg/mL or greater.
17. The method of any one of the preceding aspects, wherein the anti-C5 antibody is formulated for intravenous administration.
18. The method of any one of the preceding aspects, wherein the treatment is an administration cycle comprising a total of 26 weeks of treatment.
19. The method of any one of the preceding aspects, wherein the treatment results in terminal complement inhibition.
20. The method of any one of the preceding aspects, wherein the treatment results in a reduction of hemolysis as assessed by lactate dehydrogenase (LDH) levels compared to baseline.
21. The method of any one of the preceding aspects, wherein the treatment produces at least one therapeutic effect selected from the group consisting of: a reduction or cessation in abdominal pain, dyspnea, dysphagia, chest pain and erectile dysfunction compared to baseline.
22. The method of any one of the preceding aspects, wherein the treatment produces a shift toward normal levels of at least one hemolysis-related hematologic biomarker selected from the group consisting of: free hemoglobin, haptoglobin, reticulocyte count, PNH red blood cell (RBC) clone and D-dimer.
23. The method of any one of the preceding aspects, wherein the treatment produces a reduction in the need for blood transfusions compared to baseline.
24. The method of any one of the preceding aspects, wherein the treatment produces a reduction in major adverse vascular events (MAVEs).
25. The method of any one of the preceding aspects, wherein the treatment produces a shift toward normal levels of estimated glomerular filtration rate (eGFR) or spot urine: albumin: creatinine and plasma brain natriuretic peptide (BNP).
26. The method of any one of the preceding aspects, wherein the treatment produces a change from baseline in quality of life, assessed via the Functional Assessment of Chronic Illness Therapy (FACIT)-Fatigue Scale, version 4 and the European Organisation for Research and Treatment of Cancer, Quality of Life Questionnaire-Core 30 Scale compared to baseline.
27. The method of any one of the preceding aspects, wherein the treatment results in a reduction in free C5 concentration in the patient or reduction in red blood cell (RBC) hemolysis; particularly wherein the treatment results in free C5 concentration of 0.5 µg/mL or less and/or RBC hemolysis of 20% or less compared to an untreated patient.
28. The method of any one of the preceding aspects, wherein the treatment maintains a serum trough concentration of the anti-C5 antibody, or antigen binding fragment thereof, in the patient of at least 175 µg/mL or greater.
29. The method of any one of the preceding aspects, wherein the treatment results in improvement in at least one clinical parameter selected from (a) lactate dehydrogenase (LDH) percent change from baseline (LDH-PCHG); (b) transfusion avoidance (TA); (c) pediatric FACIT Fatigue score (FACIT); (d) hemoglobin stabilization (HGB-S); (e) free hemoglobin percent change from baseline (Free HGB-PCHG), and (f) breakthrough hemolysis (BTH) or a combination thereof; preferably wherein the treatment results in results in reduction in breakthrough hemolysis (BTH).
30. The method of any one of the preceding aspects, wherein the patient has previously been treated with eculizumab and Day 1 of the treatment is two weeks or more from the patient's last dose of eculizumab.
31. A kit for treating Paroxysmal Nocturnal Hemoglobinuria (PNH) in a human pediatric patient, the kit comprising:
   (a) a dose of an anti-C5 antibody or antigen binding fragment thereof, comprising CDR1, CDR2 and CDR3 domains of the heavy chain variable region having the sequence set forth in SEQ ID NO:12, and CDR1, CDR2 and CDR3 domains of the light chain variable region having the sequence set forth in SEQ ID NO:8; and
   (b) instructions for using the anti-C5 antibody, or antigen binding fragment thereof in the method of aspect 1 or 2.
32. An anti-C5 antibody, or antigen binding fragment thereof, comprising CDR1, CDR2 and CDR3 domains of the heavy chain variable region having the sequence set forth in SEQ ID NO:12, and CDR1, CDR2 and CDR3 domains of the light chain variable region having the sequence set forth in SEQ ID NO:8, wherein the anti-C5 antibody, or antigen binding fragment thereof, is administered:
   (a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
   (b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.
33. The antibody of aspect 32, wherein the antibody is determined to be safe, tolerable, efficacious and sufficiently non-immunogenic after multiple IV doses for use in PNH pediatric patients.

## Claims

1. An anti-C5 antibody, or antigen binding fragment thereof, for use in a method of treating a human pediatric patient with Paroxysmal Nocturnal Hemoglobinuria (PNH), wherein the anti-C5 antibody, or antigen binding fragment thereof, comprises CDR1, CDR2 and CDR3 heavy chain sequences as set forth in SEQ ID NOs:19, 18 and 3, respectively, and CDR1, CDR2 and CDR3 light chain sequences as set forth in SEQ ID NOs:4, 5 and 6, respectively, wherein the anti-C5 antibody, or antigen binding fragment thereof, is administered:
(a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

2. The anti-C5 antibody, or antigen binding fragment thereof, for use according to claim 1, wherein the anti-C5 antibody or antigen binding fragment thereof comprises a variant human Fc constant region that binds to human neonatal Fc receptor (FcRn), wherein the variant human Fc constant region comprises Met429Leu and Asn435Ser substitutions at residues corresponding to methionine 428 and asparagine 434 of a native human IgG Fc constant region, each in EU numbering.

3. The anti-C5 antibody, or antigen binding fragment thereof, for use according to claim 1 or 2, wherein the anti-C5 antibody comprises a heavy chain variable region set forth in SEQ ID NO:12 and a light chain variable region set forth in SEQ ID NO:8.

4. The anti-C5 antibody, or antigen binding fragment thereof, for use according to any one of the preceding claims, wherein the anti-C5 antibody further comprises a heavy chain constant region set forth in SEQ ID NO:13.

5. The anti-C5 antibody, or antigen binding fragment thereof, for use according to any one of the preceding claims, wherein the antibody comprises a heavy chain polypeptide comprising the amino acid sequence set forth in SEQ ID NO:14 and a light chain polypeptide comprising the amino acid sequence set forth in SEQ ID NO:11.

6. The anti-C5 antibody, or antigen binding fragment thereof, for use according to any one of the preceding claims, wherein the anti-C5 antibody binds to human C5 at pH 7.4 and 25°C with an affinity dissociation constant (K_{D}) that is in the range 0.1 nM ≤ K_{D} ≤ 1 nM.

7. The anti-C5 antibody, or antigen binding fragment thereof, for use according to any one of the preceding claims, wherein the anti-C5 antibody binds to human C5 at pH 6.0 and 25°C with a K_{D} ≥ 10 nM.

8. The anti-C5 antibody, or antigen binding fragment thereof, for use according to any one of the preceding claims, wherein the anti-C5 antibody is administered to a patient:
(i) weighing ≥ 5 to < 10 kg:
(a) once on Day 1 at a dose of 600 mg; and
(b) on Day 15 and every four weeks thereafter at a dose of 300 mg;
(ii) weighing ≥ 10 to < 20 kg:
(a) once on Day 1 at a dose of 600 mg; and
(b) on Day 15 and every four weeks thereafter at a dose of 600 mg;
(iii) weighing ≥ 20 to < 30 kg:
(a) once on Day 1 at a dose of 900 mg; and
(b) on Day 15 and every eight weeks thereafter at a dose of 2100 mg;
(iv) weighing ≥ 30 to < 40 kg:
(a) once on Day 1 at a dose of 1200 mg; and
(b) on Day 15 and every eight weeks thereafter at a dose of 2700 mg;
(v) weighing ≥ 40 to < 60 kg:
(a) once on Day 1 at a dose of 2400 mg; and
(b) on Day 15 and every eight weeks thereafter at a dose of 3000 mg;
(vi) weighing ≥ 60 to < 100 kg:
(a) once on Day 1 at a dose of 2700 mg; and
(b) on Day 15 and every eight weeks thereafter at a dose of 3300 mg; or
(vii) weighing ≥ 100 kg:
(a) once on Day 1 at a dose of 3000 mg; and
(b) on Day 15 and every eight weeks thereafter at a dose of 3600 mg.

9. The anti-C5 antibody, or antigen binding fragment thereof, for use according to any one of the preceding claims, wherein the treatment maintains a serum trough concentration of the anti-C5 antibody of 100 µg/mL or greater, or 200 µg/mL or greater.

10. The anti-C5 antibody, or antigen binding fragment thereof, for use according to any one of the preceding claims, wherein the anti-C5 antibody is formulated for intravenous administration.

11. The anti-C5 antibody, or antigen binding fragment thereof, for use according to any one of the preceding claims, wherein the treatment is an administration cycle comprising a total of 26 weeks of treatment.

12. The anti-C5 antibody, or antigen binding fragment thereof, for use according to any one of the preceding claims, wherein the treatment:
(a) results in terminal complement inhibition;
(b) results in a reduction of hemolysis as assessed by lactate dehydrogenase (LDH) levels compared to baseline;
(c) produces at least one therapeutic effect selected from the group consisting of: a reduction or cessation in abdominal pain, dyspnea, dysphagia, chest pain and erectile dysfunction compared to baseline;
(d) produces a shift toward normal levels of at least one hemolysis-related hematologic biomarker selected from the group consisting of: free hemoglobin, haptoglobin, reticulocyte count, PNH red blood cell (RBC) clone and D-dimer;
(e) produces a reduction in the need for blood transfusions compared to baseline;
(f) produces a reduction in major adverse vascular events (MAVEs);
(g) produces a shift toward normal levels of estimated glomerular filtration rate (eGFR) or spot urine: albumin: creatinine and plasma brain natriuretic peptide (BNP);
(h) produces a change from baseline in quality of life, assessed via the Functional Assessment of Chronic Illness Therapy (FACIT)-Fatigue Scale, version 4 and the European Organisation for Research and Treatment of Cancer, Quality of Life Questionnaire-Core 30 Scale compared to baseline;
(i) results in a reduction in free C5 concentration in the patient or reduction in red blood cell (RBC) hemolysis; particularly wherein the treatment results in free C5 concentration of 0.5 µg/mL or less and/or RBC hemolysis of 20% or less compared to an untreated patient;
(j) maintains a serum trough concentration of the anti-C5 antibody, or antigen binding fragment thereof, in the patient of at least 175 µg/mL or greater; and/or
(k) results in improvement in at least one clinical parameter selected from (a) lactate dehydrogenase (LDH) percent change from baseline (LDH-PCHG); (b) transfusion avoidance (TA); (c) pediatric FACIT Fatigue score (FACIT); (d) hemoglobin stabilization (HGB-S); (e) free hemoglobin percent change from baseline (Free HGB-PCHG), and (f) breakthrough hemolysis (BTH) or a combination thereof; preferably wherein the treatment results in reduction in breakthrough hemolysis (BTH).

13. The anti-C5 antibody, or antigen binding fragment thereof, for use according to any one of the preceding claims, wherein the patient has previously been treated with eculizumab and Day 1 of the treatment is two weeks or more from the patient's last dose of eculizumab.

14. The anti-C5 antibody, or antigen binding fragment thereof, for use according to any one of the preceding claims, wherein the anti-C5 antibody, or antigen binding fragment thereof, comprises CDR1, CDR2 and CDR3 domains of the heavy chain variable region having the sequence set forth in SEQ ID NO: 12, and CDR1, CDR2 and CDR3 domains of the light chain variable region having the sequence set forth in SEQ ID NO:8, wherein the method comprises administering the anti-C5 antibody, or antigen binding fragment thereof:
(a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

15. The anti-C5 antibody, or antigen binding fragment thereof, for use according to claim 14, wherein the antibody is determined to be safe, tolerable, efficacious and sufficiently non-immunogenic after multiple IV doses for use in PNH pediatric patients.
